# EUROPEAN PATENT APPLICATION

(11) **EP 0 921 190 A2**
(43) Date of publication of application: **09.06.1999**
(21) Application number: 98403046.0
(22) Date of filing: 04.12.1998
(51) Int. Cl.: C12N 15/01, C12N 1/19, A21D 8/04

(54) **Frozen dough-resistant and high-sugar dough-resistant, practical baker's yeast**

(30) Priority: 08.12.1997 JP 35201697
(71) Applicant: National Food Research Institute, Ministry Of Agriculture, Forestry and Fisheries, Tsukuba-shi, Ibaraki-ken (JP); ORIENTAL YEAST CO., LTD., Tokyo (JP)
(72) Inventor: Takano, Hiroyuki, Tsukuba-shi, Ibaraki-ken (JP); Shima, Jyun, Tsukuba-shi, Ibaraki-ken (JP); Iyo, Chie, Tsukuba-shi, Ibaraki-ken (JP); Mori, Katumi, Tsukuba-shi, Ibaraki-ken (JP); Suzuki, Yasuo, Inba-gun, Chiba-ken (JP); Nakajima, Ryoichi, Tokyo (JP); Watanabe, Hajime, Wakou-shi, Saitama-ken (JP)
(74) Representative: Clisci, Serge

(57) **Abstract**

The invention relates to a diploid or higher polyploid, practical baker's yeast with good frozen dough resistance and good high-sugar dough resistance. This is produced through mating with one or more ATH1 gene-disrupted, haploid yeasts as produced through gene manipulation of disrupting the ATH1 gene in a haploid yeast of which the diploid is practical baker's yeast. The practical baker's yeasts of the invention has a reduced ability of trehalose degradation during fermentation even when used in frozen dough or in high-sugar dough, and is well resistant to long-term freeze-storage when used not only in low-sugar dough but also in high-sugar dough.

## Description

### Detailed Description of the Invention:

### Technical Field of the Invention:

The present invention relates to extremely excellent, frozen dough-resistant practical baker's yeast and high-sugar dough-resistant practical baker's yeast. Conventional frozen dough-resistant baker's yeast and high-sugar dough-resistant baker's yeast have heretofore been produced, over which the frozen dough-resistant practical baker's yeast and the high-sugar dough-resistant practical baker's yeast of the invention are significantly excellent. Frozen dough as produced through the process of preparing dough with the frozen dough-resistant baker's yeast of the invention followed by incubating and freezing it is resistant to long-term frozen storage of 2 weeks or longer, from which is produced good bread. The long-term stored, frozen dough gives, when thawed and baked, bread which is comparable to or better than that from the frozen dough as prepared with the conventional frozen dough-resistant baker's yeast and stored long. Specifically, in the invention in which the ATH1 gene in practical baker's yeast having various excellent characteristics but not having resistance to frozen dough is inactivated, it has become possible to make the practical baker's yeast have frozen dough resistance that is comparable to or higher then that of ordinary commercially-available, frozen dough-resistant baker's yeast, and even to make it have high-sugar dough resistance. Therefore, considering the fact that frozen dough generally has a high sugar content, the frozen dough-resistant/high-sugar dough-resistant, practical baker's yeast of the invention greatly contributes to developments in the frozen dough industry.

### Prior Art:

Regarding the frozen dough resistwce of yeast of Saccharomyces cerevisiae, a technique of gene manipulation to ensure the accumulation of trehalose in the yeast was reported by Helmut Holzer et al. of the Freiburg University (see J.B.C., Vol. 268, No. 7, 1993). This technique was applied to practical strains, and a patent application was filed for "Frozen Dough-Resistant, Practical Baker's Yeast" (Japanese Patent Application No. Hei-8-297886). The report and the patent application relate to the NTH1 gene (neutral trehalase gene) of that yeast.

After that, the same attempt was made on a gene ATH1 (acidic trehalase gene). This gene was cloned and analyzed by Momoca Destruelle, et al. of the Freiburg University (see Yeast, Vol. 11, 1995). John Kim, et al. of the California University found that the trehalose accumulation in an ATH1 gene-disrupted strain is significantly increased, and they suggested the practical use of that strain in baker's yeast because of its freezing resistance and drying resistance (see Applied and Environmental Microbiology, Vol. 62, No. 5, 1996). However, they used laboratory strains in their studies, and nothing is suggested for the usefulness of the ATH1 gene disruption in practical baker's yeast. In other words, their studies were not confirmed in the practical baking industry, and at present, the industrial usefulness of the ATH1 gene-disrupted strain is not confirmed at all.

The matter to be specifically noted herein is that laboratory strains and practical baker's yeast strains greatly differ in their behavior. For example, as so mentioned in the report of Takano, et al. of the National Food Research Institude, Ministry of Agriculture, Forestry and Fisheries of Japan (red in the Meeting of the Food Engineering Society of Japan in 1997), and in the specification of Japanese Patent Application Hei-8-297896 for "Frozen Dough-Resistant, Practical Baker's Yeast", laboratory strains and practical baker's yeast strains greatly differ in the effect of NTH1 gene disruption thereon. Naturally, therefore, it is anticipated that laboratory strains and practical baker's yeast strains will greatly differ in the effect of ATH1 gene disruption thereon, and it is rather anticipated that the effect on laboratory strains could not be exhibited on practical baker's yeast strains.

The neutral trehalase encoded by the NTH1 gene exists in cytoplasm, and its action has been clarified; while the acidic trehalase encoded by the ATH1 gene exists in vacuole, and its action still remains no more than speculation. Accordingly, the two could not be discussed on the same level, and it must be said that the matter as to whether or not the ATH1 gene disruption could be effective even in practical strains like the NTH1 gene disruption therein is still quite unknown.

When practical baker's yeast is improved to thereby make it have specific capabilities in conventional breeding methods comprising mating different types of strains, their intrinsic and favorable properties are often sacrificed.

Where the yeast of Saccharomyces cerevisiae is used as practical baker's yeast, its source shall be a species of Saccharomyces cerevisiae, and, in addition, the yeast must satisfy various requirements needed in its practical use. For example, the indispensable requirements for the yeast are as follows: 1. In order to cultivate it in an industrial scale, the yeast must grow well in blackstrap molasses media that are generally used for mass-cultivating baker's yeast (that is, the growth rate of the yeast is high, and the yield thereof is large), the baker's yeast having grown in the media must be efficiently separated therefrom, and the efficiency in the dehydrating step in the process of forming the thus-separated baker's yeast into commercial products must be high (in that step, used is a specific device of a so-called dehydrator). 2. The products could keep the yeast activity (dough-expanding activity) during long-term cold storage (generally, for a few weeks), they are hardly softened during the storage, they are white without adsorbing blackstrap molasses. Apart from those, the yeast is required to have capabilities compatible with various baking methods.

For the reasons noted above, there is almost no possibility that laboratory strains not subjected to the screening for practical baker's yeast could be directly used as those for practical baker's yeast. In general, it is unusual that the requirements noted above are rarely governed by one gene but are often defined by a plurality of genes. In addition, there are known few studies relating to the relationship between the properties of practical baker's yeast and the genes constituting tie yeast.

Being different from laboratory strains, most strains for baker's yeast are polyploidal and hardly form spores. Tnerefore, even the application of the breeding method of mating/spore separation that is based on classic breeding systems and is generally applied to laboratory strains, to baker's yeast strains is extremely difficult. Good baker's yeast shall exclusively rely on the screening of spontaneous mutants from ordinary strains that have been practically used in the art for many years, or on the screening thereof from the natural world. Gunge, et al. first applied a classic breeding method to baker's yeast in their rare-mating method (see Genetics, Vol. 70, 1972), and after that, one example of practical application of the method was first descried in the specification of a patent application filed in 1982 for "Saccharomyces species FD612" (see Japanese Patent Publication Hei-1-16155).

### Problems to be Solved by the Invention:

However, as so mentioned hereinabove, one necessary property of baker's yeast is governed by a large number of genes. Therefore, where strains having good properties of baker's yeast are transformed to thereby make them have specific capabilities according to classic breeding methods, their favorable and intrinsic characters are often lost. For example, when baker's yeast capable of producing good bread taste is bred through mating/spore separation for making it have freezing resistance or high-sugar dough resistance, the ability of the yeast to produce good bread taste is often attenuated. In that case, an extremely large number of strains of the posterity must be screened for the strains capable of producing relatively good bread taste. The subject matter of the present invention is to make baker's yeast strain have frozen dough resistance or high-sugar dough resistance while leaving the strain to still keep its favorable and intrinsic properties.

In ordinary gene manipulation, used is haploid strains. However, most baker's yeasts now used in Japan are diploid. This is because, as compared with haploid ones, diploid and higher polyploid strains are larger in size and grow better, and therefore they are more easily satisfy the practical requirements noted above. The strain group described in the patent application for "Frozen Dough-Resistant Practical Baker's Yeast" (Japanese Patent Application Hei-8-297886) was developed for the purpose of compensating for the gap. The strains described were previously so tested that they could exhibit various excellent properties when they are mated in suitable combination to give diploids.

The conventional gene manipulation of disrupting the ATH1 gene (acidic trehalase gene) in yeast may produce the increase in the amount of trehalose to be formed in the resulting yeast, but frozen dough-resistant, practical baker's yeast capable of finally giving delicious bread could not be obtained as yet. Given this situation, one object of the invention is to construct frozen dough-resistant, practical baker's yeast capable of finally giving delicious bread, to produce excellent frozen dough, and to produce delicious bread by thawing, fermenting and baking the frozen dough.

On the other hand, it is known that the dough-expanding force of practical baker's yeast generally lowers when the sugar content of dough is larger than 5 % relative to the wheat flour content thereof. In practical formula of dough for commercial bread products, the sugar concentration may be 40 % or more. Yeast of which the fermenting power is lowered little even in dough having a high sugar concentration (high-sugar dough) is herein referred to as high-sugar dough-resistant yeast. High-sugar dough-resistant, practical baker's yeast is known. However, the intracellular substances in the yeast, which could be the index for the high-sugar dough resistance of the yeast, are not analyzed as yet, and even though analyzed, there is known no study report in which the analyzed data are associated with the bread-producing capabilities of the yeast. Given this situation, another object of the invention is to disrupt the ATH1 gene in a strain thereby increasing the intracellular trehalose content of the resulting strain and increasing the intracellular osmotic pressure in the strain for positively constructing high-sugar dough-resistant, practical baker's yeast, to produce excellent high-sugar dough, and to produce delicious bread (sweetened buns, etc.) from the high-sugar dough.

### Means for Solving the Problems:

Even though freezing-resistant yeast could be constructed trough ATH1 gene disruption, frozen dough-resistant practical baker's yeast or high-sugar dough-resistant practical baker's yeast capable of producing delicious bread could not be obtained as yet. We, the present inventors desired to modify practical baker's yeast having excellent properties but not having resistance to frozen dough or to high-sugar dough into frozen dough-resistant/high-sugar dough-resistant, practical baker's yeast still having its original excellent properties and additionally having frozen dough resistance/high-sugar dough resistance that is comparable to or higher than that of ordinary, commercially-available freezing-resistant yeast. For this purpose, we analyzed in detail starting yeast strains, frozen dough or high-sugar dough, and even final bread from the dough in various experiments and, as a result, have completed the invention.

The invention relates to a set of ATH1 gene-disrupted, haploid yeasts as produced through gene manipulation of disrupting the ATH1 gene in a set of haploid yeasts of which the original hybridized diploid is practical baker's yeast.

The invention also relates to a diploid or higher polyploid, frozen dough-resistant/high-sugar dough-resistant, practical baker's yeast as produced through mating with one or more ATH1 gene-disrupted, haploid yeasts produced through gene manipulation of disrupting the ATH1 gene in a haploid yeast of which the diploid is practical baker's yeast.

At least one haploid yeast to be used in that mating most be the ATH1 gene-disrupted, haploid yeast, but may be combined with yeasts with no gene disruption. The invention further relates to frozen dough-resistant/high-sugar dough-resistant, practical baker's yeast-containing, frozen or high-sugar dough, as produced by preparing dough with a diploid or higher polyploid, frozen dough-resistant/high-sugar dough-resistant, practical baker's yeast that is produced through mating with one or more ATH1 gene-disrupted, haploid yeasts produced through gene manipulation of disrupting the ATH1 gene in a haploid yeast of which the diploid is practical baker's yeast. (1) The non-frozen dough is then incubated and thereafter frozen to give the frozen dough. Optionally, the frozen dough is thawed, fermented and baked to give delicious bread. (2) The high-sugar dough is directly fermented and baked also to give delicious bread.

### Brief Description of the Drawings :

Fig. 1 shows a process of constructing a vector for ATH1 gene disruption.

Fig. 2 is a conceptual view showing ATH1 gene disruption.

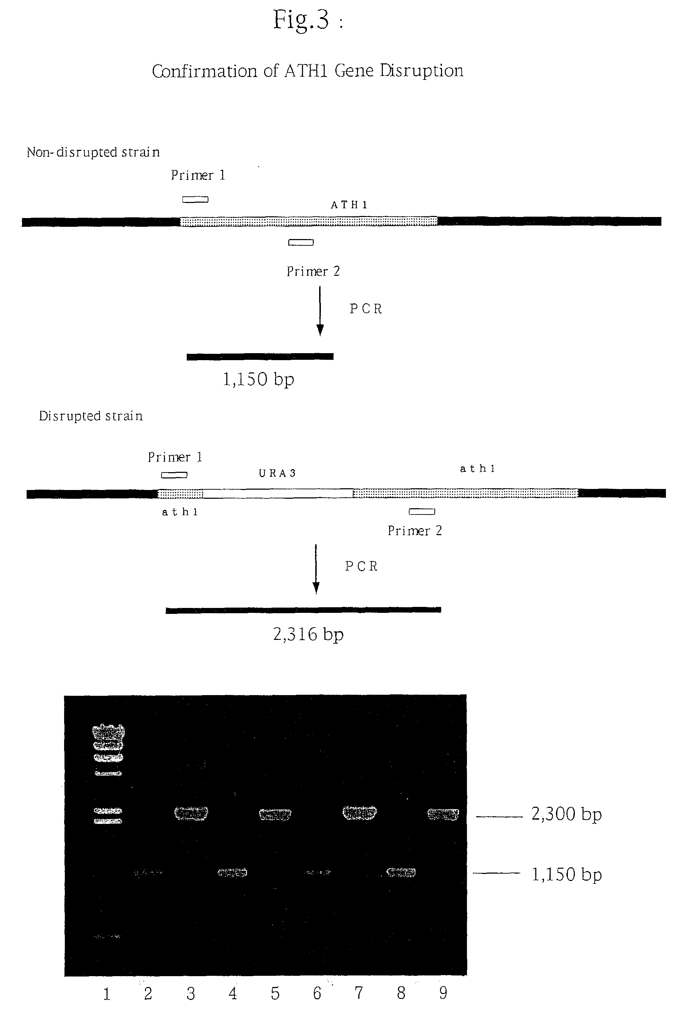

Fig. 3 shows the confirmation of ATH1 gene disruption, in which the lane 1 indicates a molecular weight marker, the lanes 2, 4, 6 and 8 indicate PCR products of DNA derived from strains 7, 21, 18 and 19, respectively, and the lanes 3, 5, 7 and 9 indicate PCR products of DNA derived from strains 7dA, 21dA, 18dA and 19dA, respectively.

Fig. 4(A) shows the time-dependent variation in the acidic trehalase activity of each strain of T118 and A318 in culture; and Fig. 4(B) shows the time-dependent variation in the intracellular trehalose content of each strain of T118 and A318 in culture.

Fig. 5(A) shows the time-dependent variation in the acidic trehalase activity of each strain of T128 and A328 in culture; and Fig. 5(B) shows the time-dependent variation in the intracellular trehalose content of each strain of T128 and A328.

Fig. 6 shows the time-dependent variation in the trehalose content of each strain of T118 and A318 in fermentation.

Fig. 7 shows the time-dependent variation in the trehalose content of each strain of T128 and A328 in fermentation.

Fig. 8 shows the time-dependent variation in the trehalose content of each strain of commercially-available yeasts in fermentation.

Fig. 9 shows the data of the gaseous volume of each low-sugar dough sample as obtained through fermography, for which each dough sample comprising a different yeast was incubated for 60 minutes, then frozen and stored for 1 to 3 weeks, thawed, and thereafter subjected to fermography at 30°C for 120 minutes.

Fig. 10 shows the data of the gaseous volume of high-sugar dough samples, which were obtained in the same manner as in Fig. 9.

### Modes of Carrying out the Invention:

### 1. (Screening for haploid yeasts of which the diploids are practical baker's yeasts, and genetic marking)

In the invention, the screening of yeast strains for those to be subjected to gene manipulation is indispensable. First are selected haploid yeast strains, which must be identified as to whether they are a-type ones or α-type ones. Where a selected haploid yeast could be conjugated with a previously prepared α-type haploid yeast within a few hours in the culture of the two in a ratio of 1/1 and where the conjugation could be confirmed with a microscope, the haploid yeast is identified as an a-type one. On the other hand, where the conjugation of a selected haploid yeast with a previously prepared a-type haploid yeast in the culture of the two in a ratio of 1/1 could be confirmed, the haploid yeast is identified as an α-type one. In laboratory experiments, the thus-identified haploid yeasts could be subjected to the next step of genetic marking. In constructing practical baker's yeasts, however, a step of screening the identified haploid yeasts is indispensable prior to the genetic marking step. The screening step may be effected in the manner mentioned below.

An a-type or α-type haploid yeast is mated will an α-type or a-type haploid to construct an a/α-type diploid yeast. Then, in the first stage, the resulting diploid strains are screened in a mass-cultivation test such as that mentioned hereinabove, which is based on the possibility of industrial production of the strains. Next, in the second stage, the strains thus cultivated and screened in the previous test are further screened for those that satisfy the requirements of commercial products also mentioned hereinabove. Finally, the strains thus passed the two-stage screening are cultivated, and the thus-cultivated strains are used in producing different types of bread. In this final step, the strains with which excellent bread samples were produced are selected. Thus, the yeasts used in preparing the excellent bread samples are known, and they are determined to be haploid yeasts to be subjected to gene manipulation.

There are various types of bread, including, for example, loaves, rolls, croissants, French bread and rolls, and buns, for all of which diploid yeasts as constructed from various haploid yeasts are tested and tasted. Depending on the type of the haploid yeasts to be mated, as to whether they are a-type ones or α-type ones, the characteristics of the bread to be prepared by baking frozen dough or high-sugar dough that comprises the mated diploid yeast greatly vary. Therefore, the screening of the suitable haploid yeast to be subjected to gene manipulation is extremely difficult. However, in order to obtain the intended, frozen dough-resistant or high-sugar dough-resistant, practical baker's yeast, this screening step is indispensable.

Gene manipulation of yeast requires genetic markers. Practical baker's yeast has no marker, and where it is subjected to gene manipulation, employable are markers except those for the genes of the yeast, such as chemical-resistant markers and the like, which, however, are unfavorable from the viewpoint of the safety in the gene manipulation. Haploid strains to be subjected to gene manipulation within the range of their self-cloning require genetic markers. In that case, if the yeast to be processed is mutated with chemicals or the like, it will lose its intrinsic favorable properties. For this, therefore, it is desirable to employ a screening method for spontaneous mutants. For example, as the marker genes, preferred are URA3, LYS2, ADE2 and the like for easy screening for the intended defective strains.

To introduce a gene marker, ura3 (URA3-defective strain) into a haploid strain, cells of the strain are screened in a 5-fluoro-orotic acid-containing medium. Briefly, cells of a haploid strain are cultivated in an YPD liquid medium, centrifuged, and washed with a physiological saline solution. About 10⁸ cells thus cultivated are applied onto a 5-fluoro-orotic acid-containing medium (0.7 % YEAST NITROGEN BASE (DIFCO), 2 % glucose, 0.1 % 5-fluoro-orotic acid, 0.05 % uracil, 2 % agar) and cultivated thereon at 30°C for 3 days, and the cells growing on the medium to give colonies thereon are selected. The cells having grown on the medium do not have URA3 gene, as having been spontaneously mutated. Such URA3-defective cells are obtained at a frequency of one cell per 10⁶ to 10⁷ cells. Those URA3-defective cells could not grow on an uracil-free medium, but could grow thereon only after having been transformed with an URA3-containing plasmid, such as YCp50 or the like. Therefore, through the transformation of those cells, the defect of URA3 therein can be confirmed.

### 2. (Cloning of acidic trehalase gene)

Cloning of acidic trehalase gene ATH1 may be attained for example, in `an ordinary shot-gun cloning method based on the fact that ATH1 gene-defective strains could not grow in a medium comprising trehalose as only one carbon source. However, since the full-length base sequence of the chromosome of the yeast of Saccharomyces cerevisiae was already known, the cloning of the acidic trehalase gene ATH1 of which the sequence was already clarified is generally effected through PCR (polymerase chain reaction). For inactivating (disrupting) the gene, cloning of the full-length gene is unnecessary, and a partial region of the gene may be cloned.

### 3. (Construction of vector for ATH1 gene disruption, and disruption of ATH1 gene)

The object of the disruption of the ATH1 gene in haploid yeasts is to prevent the ATH1 gene from being expressed in the yeasts to give an acidic trehalase which decomposes trehalose. For this, therefore, all or a part of the gene sequence of the ATH1 gene is deleted. Preferably, in the invention, the ATH1 gene in a haploid yeast is completely replaced with URA3, or URA3 is inserted into the ATH1 gene thereby substantially inactivating the ATH1 gene for disrupting the gene in the yeast.

First, a part of the amino acid-coding region of the ATH1 gene of Sequence Number 3 in the Sequence Listing is, after having been amplified through PCR, inserted into an E. coli vector, such as pGEM-T, then the full-length URA3 gene of Sequence Number 4 is inserted into the partial region of the ATH1 gene in the vector. The resulting plasmid is proliferated in E. coli cells. From this plasmid, cleaved out is only the DNA fragment of ATH1 gene (former half) - URA3 - ATH1 gene (latter half). With the thus-isolated DNA fragment, thereafter transformed is a haploid yeast, of which the diploid is a practical baker's yeast, by a lithium acetate method. The DNA fragment, ATH1 gene (former half) - URA3 - ATH1 gene (latter half) in the haploid yeast is site-specifically recombined with the ATH1 gene in the yeast, whereby the ATH1 gene is completely divided into two, its former half and latter half, via URA3 therebetween, resulting in that the gene is completely disrupted and is substantially inactivated. Sequence Number 5 indicates the amino acid-coding region of the ATH1 gene as cleaved with URA3.

### 4. (Formation of Diploid)

The ATH1 gene-disrupted haploid yeast obtained herein is either an a-type or α-type one, while having such necessary properties that its diploid yeast can be a practical baker's yeast. In other words, only the ATH1 gene is disrupted in the haploid yeast through the gene disruption, while the other genes in the resulting ATH1 gene-disrupted haploid yeast are not changed at all and still maintain their intrinsic properties. In the invention, one or more ATH1 gene-disrupted haploid yeasts as prepared through the process of disrupting the ATH1 gene of a haploid yeast, of which the diploid is a practical baker's yeast, are mated with any other haploid yeasts to give diploid or higher polyploid, frozen dough-resistant/high-sugar dough-resistant, practical baker's yeasts.

### Examples:

### Example 1:

### (Screening for haploid yeast of which the diploid is practical baker's yeast)

25 stock cultures of wild haploid yeasts were identified as to whether they are a-type ones or α-type ones, and all of these were tested to know as to whether or not their diploids could be practical baker's yeasts. As a result of the test, 8 strains as in Table 1 were selected. These 8 strains were subjected to ATH1 gene disruption according to fine method mentioned below, by which the ATH1 gene existing therein was disrupted. Before and after the gene disruption, the acidic trehalase activity of each strain was measured.

The data obtained are shown in Table 1, from which it was confirmed that the acidic trehalase activity of the ATH1 gene-disrupted strains was significantly lowered. That is, the data indicate the disruption of the ATH1 gene in those strains.

**Table 1**

| Comparison between the ATH activity of haploid yeast strain (wild strain), of which the diploid is practical baker's yeast, and that of ATH1 gene-disrupted strain | |
|---|---|
| Sample | Acidic Trehalase Activity (mU/mg protein) |
| 2 (ATH1) | 6.13 |
| 2dA (ath1) | 0.20 |
| 7 (ATH1) | 4.96 |
| 7dA (ath1) | 0.25 |
| 14 (ATH1) | 8.55 |
| 14dA (ath1) | 0.18 |
| 21 (ATH1) | 5.14 |
| 21dA (ath1) | 1.31 |
| 12 (ATH1) | 9.01 |
| 12dA (ath1) | 0.82 |
| 13 (ATH1) | 6.22 |
| 13dA (ath1) | 0.06 |
| 18 (ATH1) | 8.55 |
| 18dA (ath1) | 1.36 |
| 19 (ATH1) | 9.06 |
| 19dA (ath1) | 0.67 |

### (Cloning of ATH1 gene)

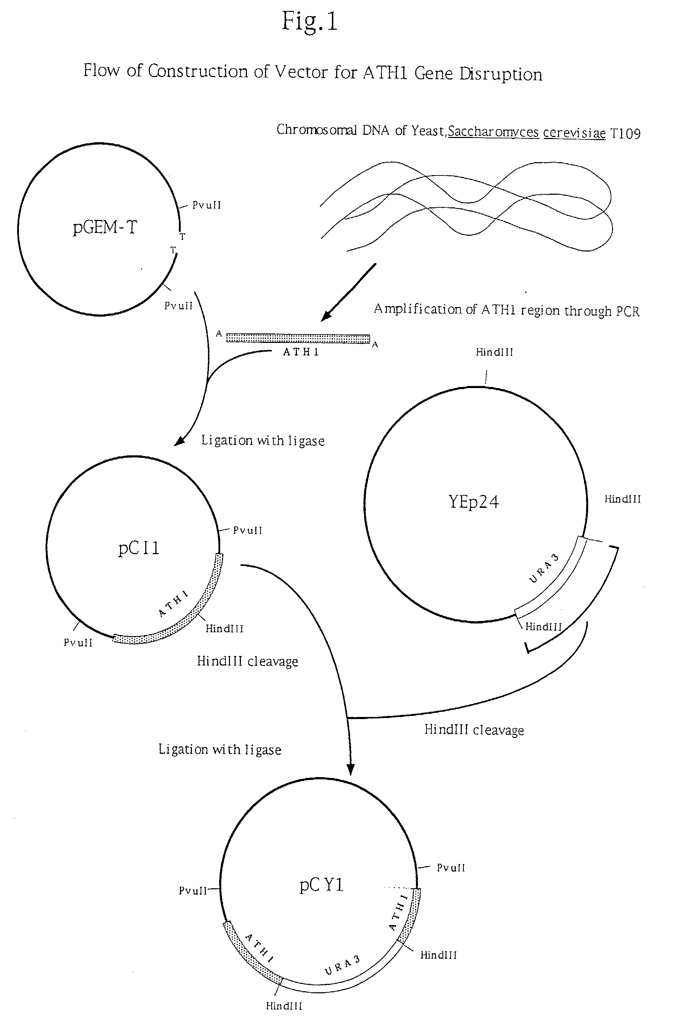

A partial sequence of ATH1 gene was cloned, using a Promega's pGEM-T vector system. The outline of the cloning is shown in Fig. 1.

Precisely, based on the known base sequence of ATH1 gene (see Yeast, Vol. 11, 1995), 20 bp primers of Sequence Number 1 and Sequence Number 2 were designed in an ordinary manner. A chromosomal DNA was extracted from the yeast of Saccharomyces cerevisiae T019, and purified. Using this DNA as the template, along with the synthetic DNA primers of Sequence Numbers 1 and 2 noted above, a partial sequence of 1,150 base pairs between the 481st base and the 1,630th base in the coding region for ATH1 gene was amplified through PCR. Next, the thus-amplified DNA fragment was purified in an ordinary manner, and ligated with a pGEM-T vector by the use of a T4 DNA ligase. Thus was obtained a plasmid pCI1. This plasmid pCI1 was introduced into cells of E. coli (transformation) in an ordinary manner, amplified, and purified. Next, the restriction enzyme HindIII-recognition site, which is only one in the ATH1 gene partial sequence in the purified plasmid, was cleaved with the enzyme HindIII, On the other hand, a known plasmid YEp24 having URA3 gene was cleaved with the enzyme HindIII to isolate 1,166 bp DNA fragment containing the full-length of URA3 gene from it. After having been purified in an ordinary manner, the DNA fragment of URA3 gene was ligated with the HindIII-cleaved pCI1 to construct a plasmid pCY1 for ATH1 gene disruption.

### (Formation of ATH1 gene-disrupted haploid)

According to the method mentioned hereinabove, an a-type haploid yeast (wild strain) was processed to be a strain of ura3, and then transformed with the plasmid pCY1 (this is for ATH1 gene disruption) that had been cleaved with a restriction enzyme PvuII, according to a lithium acetate process. The non-transformed strain requires uracil in its growth, and the transformant was identified by its character not reguiring uracil in its growth.

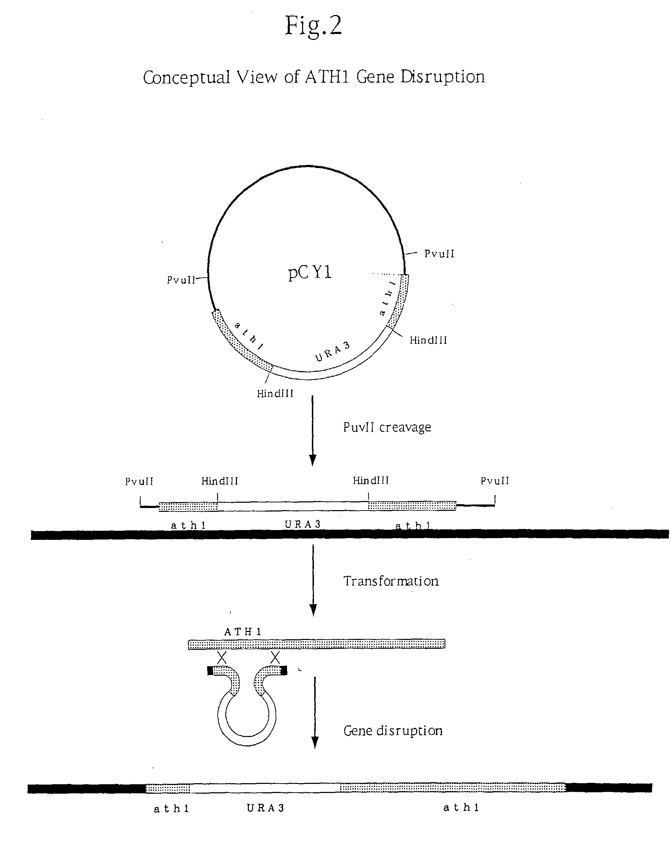

In the same manner, an α-type haploid yeast (wild strain) was processed to be a strain of ura3 according to the method mentioned hereinabove. On the other hand, the plasmid pCY1 for ATH1 gene disruption was cleaved with a restriction enzyme PvuII, and the DNA fragment comprising the ATH1 partial sequence and URA3 was isolated and purified. The ura3 strain was transformed with this DNA according to a lithium acetate process. The non-transformed strain requires uracil in its growth, and the transformant was identified by its character not requiring uracil in its growth. (Fig. 2).

### (Confirmation of ATH1 gene disruption)

The ATH1 gene disruption was theoretically confirmed through PCR.

Precisely, the total chromosomal DNA. was extracted from both the wild strain and the gene-disrupted strain, and each DNA was amplified with the primers of Sequence Number 1 and Sequence Number 2, and the thus-amplified DNAs were compared with each other with respect to their length. The data are shown in Fig. 2. Theoretically, the DNA fragment from the wild strain is amplified to have 1,150 bp, while that from the gene-disrupted strain is to have 2,316 bp. The theoretical data correspond to the data measured, which supports the gene disruption.

### (Confirmation of ATH1 gene inactivation)

To confirm the ATH1 gene disruption, the acidic trehalase activity of both wild strains (ATH1, in which the ATH1 gene is not disrupted) and ATH1 gene-disrupted strains (ath1) was measured. Precisely, 18-φ test tubes each charged with 5ml of an YPD medium (1 % yeast extract, 2 % peptone, 2 % glucose) were prepared, in which were separately planted one platinum loop of cells of each wild strain and one platinum loop of cells of each ATH1 gene-disrupted strain, and cultivated therein at 30°C for 48 hours with shaking. The acidic trehalase activity of the thus-cultivated cells was measured in an ordinary manner. The data obtained are in Table 1. The acidic trehalase activity of the gene-disrupted strains was obviously lowered, as compared with that of the non-disrupted wild strains, and was nearly 0 (zero). This supports the ATH1 gene inactivation in the disrupted strains.

### Example 2:

### (Mating of a-type haploid yeast and α-type haploid yeast)

The mating matrix in Table 2 shows various combinations of wild strains and pCY1-processed, ATH1 gene-disrupted strains.

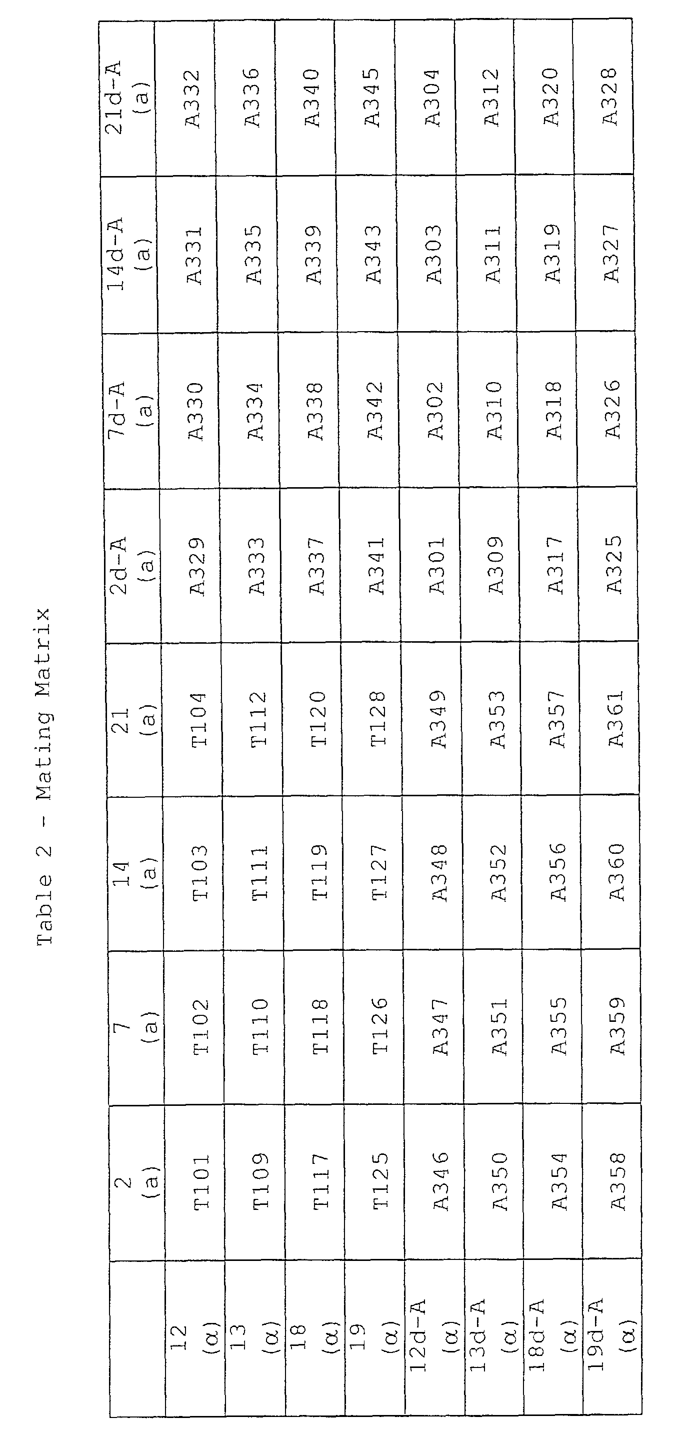

In Table 2, the strains in the uppermost row are all a-type ones, while those in the leftmost column are all α-type ones. In this, the strains with "d-A" are gene-disrupted ones as processed with pCY1; while those with no "d-A" are wild strains. Each one in the uppermost row was mated with each one in the leftmost column to obtain 64 diploid yeasts, T101 through T128 and A301 through A361, in all as in Table 2.

The mating was effected as follows: First, a pair of a-type strain and α-type strain were separately cultivated and proliferated in YPD media at 30°C for one day. Nearly the same number of the proliferated cells of the both strains were put into a fresh YPD medium and further cultivated therein at 30°C for 12 hours. Then, a suitable amount of the thus-cultivated cells were applied onto a YPD-agar medium, and cultivated thereon at 30°C for one or two days. Relatively large colonies formed were taken out. It was confirmed that the cells in those colonies have no conjugating ability and that they are larger than the haploid cells through microscopic observation. Thus, the formation of diploid yeast cells was confirmed.

### Example 3:

### (Cultivation of diploid, frozen dough-resistant/high-sugar dough-resistant, practical yeast strains)

Diploid, frozen dough-resistant/high-sugar dough-resistant, practical yeast strains were cultivated in 30-liter jar fermenters under the condition mentioned below.

### 30-Liter jar culture

| | Seed Culture | Main Culture |
|---|---|---|
| Saccharide (in terms of sucrose) | 1035 g | 1400 g |
| Urea | 103 g | 140 g |
| Monosodium phosphate dihydrate | 20.7 g | 28 g |
| Seed yeast (wet) | 20 g(*1) | 420 g(*2) |
| 30-Liter jar: | | |
| Maker: Oriental Bioservice KK | | |
| Name: Fermenter Control System MC-10 | | |
| Volume: 30 liters | | |
| Revolution of stirrer: 600 rpm | | |
| Aeration: 16 liters/min | | |

| | | |
|---|---|---|
| *1: One platinum loop of yeast cells was planted in a 1-liter Sakaguchi flask charged with 250 ml of a YPD medium, and cultivated therein at 30°C for 2 days. The cells of four flasks were used as the seed cells. | | |
| *2: The cells grown in the seed culture were taken out through centrifugation, and washed with deionized water. A part of those cells were used. | | |

All the tested strains gave an yield of from 120 to 140 %, relative to the saccharide used, of the yield given by the commercially-available baker's yeast strain as cultivated in the same manner. The data verify that those strains can be cultivated on an industrial scale, and that the ATH1 gene disruption in those strains did not interfere with the requirements including the cell growability and the storage stability necessary to practical baker's yeast.

### (Time-dependent variation in trehalose content and trehalase activity of strain in liquid culture)

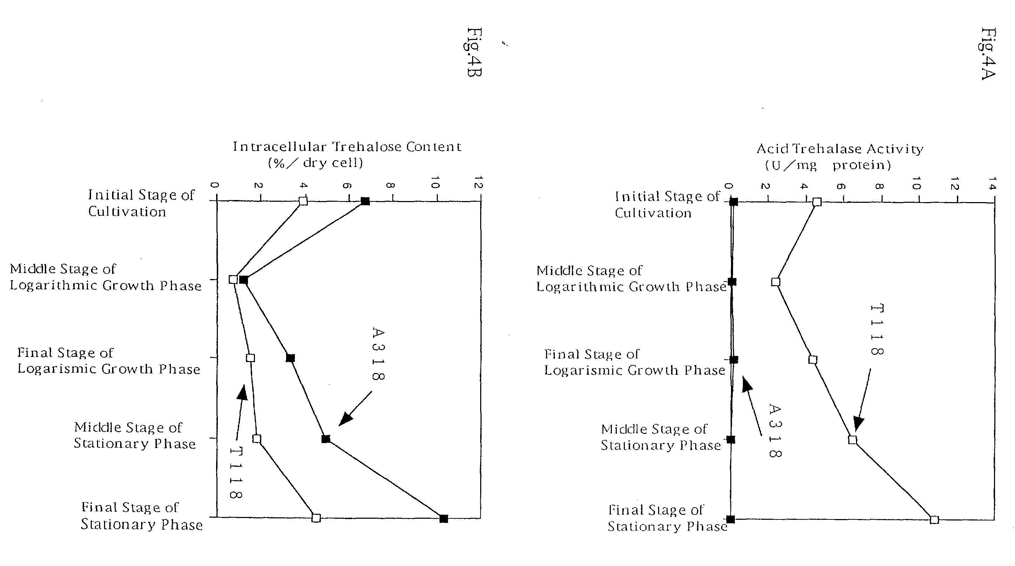

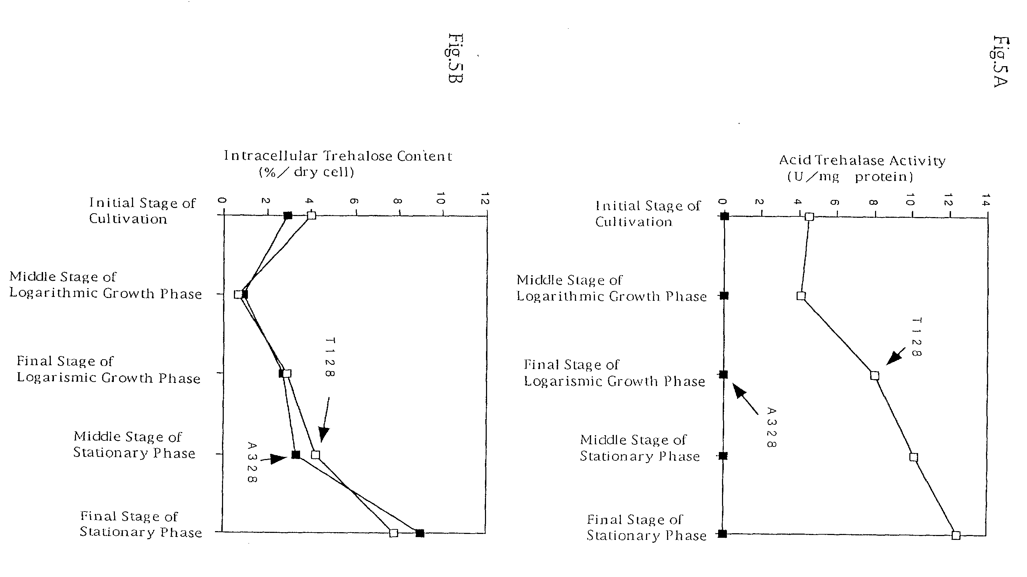

The cultivation process employed herein could be divided into two stages, one being a logarithmic growth phase stage and the other being a stationary phase stage. Of 64 strains in Table 2 above, cells of four strains of T118, T128 and their ATH1 gene-disrupted strains, A318 and A328 being cultivated under the condition noted above were sampled in the initial, middle and last stages in each phase, and their acidic trehalase activity (A) and trehalose content (B) were measured. The data obtained are shown in Fig. 4 and Fig. 5. As in these, the acidic trehalase activity of each gene-disrupted strain was nearly 0 in every stage in culture. The data further verify the ATH1 gene inactivation in those gene-disrupted strains.

Strains T118 and T128 were named Saccharomyces cerevisiae T118 and Saccharomyces cerevisiae T128, respectively, and were deposited on September 3, 1997 in the international depository authority, the National Institute of Bioscience and Human-Technology, Agency of Industrial Science and Technology of Japan, for FERM BP-6096 and FERM BP-6097, respectively, according to the Budapest Treaty. Their ATH1 gene-disrupted strains A318 and A328 were named Saccharomyces cerevisiae A318 and Saccharomyces cerevisiae A328, respectively, and were deposited on August 8, 1997 in the same international depository authority for FERM BP-6039 and FERM BP-6040, respectively, according to the Budapest Treaty.

Comparing the wild strain T118 and its ATH1 gene-disrupted strain A318, it is known that the gene disruption obviously resulted in the increase in the trehalose content of the gene-disrupted strain in the final stage of the cell culture, as in the literature noted above, and the trehalose content of the gene-disrupted strain was about 2 times that of the wild strain. On the contrary, when the wild strain T128 is compared with its ATH1 gene-disrupted strain A328, it is known that the acidic trehalase activity of the ATH1 gene-disrupted strain A328 was lowered to nearly 0 (zero) but that its trehalose content in the final stage of the cell culture was nearly the same as that of the wild strain. In the test of practical baker's yeast strains for their capabilities, generally used are the cells having been cultivated in feeding culture as a simulation of industrial-scale culture, for example, in the manner mentioned above. In the test, the intracellular trehalose content could be 8 +/- 2 % or so, relative to the dry cells, and the trehalose content of the wild strain T118 somewhat differs from that of practical baker's yeast strain generally used in the art. As so mentioned hereinabove, most laboratory yeast strains could not apply to the feeding culture system. If, however, they are intentionally cultivated in the feeding culture system test, while neglecting the production efficiency in the test, their trehalose content will fall between 2 and 4 % or so, which significantly differs from the trehalose content of practical strains. In the test of other practical baker's yeast strains shown in Table 2, the ATH1 gene disruption in the wild stratus, of which the trehalose content in the final stage of their culture is nearly the same as that of laboratory strains, like T118, resulted in significant increase in the amount of trehalose produced in the gene-disrupted strains, but the effect of the ATH1 gene disruption in the other wild strains of which the trehalose production is nearly the same as that of practical baker's yeast generally used in the art was only small. In this point, the laboratory strains shall be differentiated from the practical baker's yeast strain.

### (Time-dependent variation in trehalose content of strain in liquid culture)

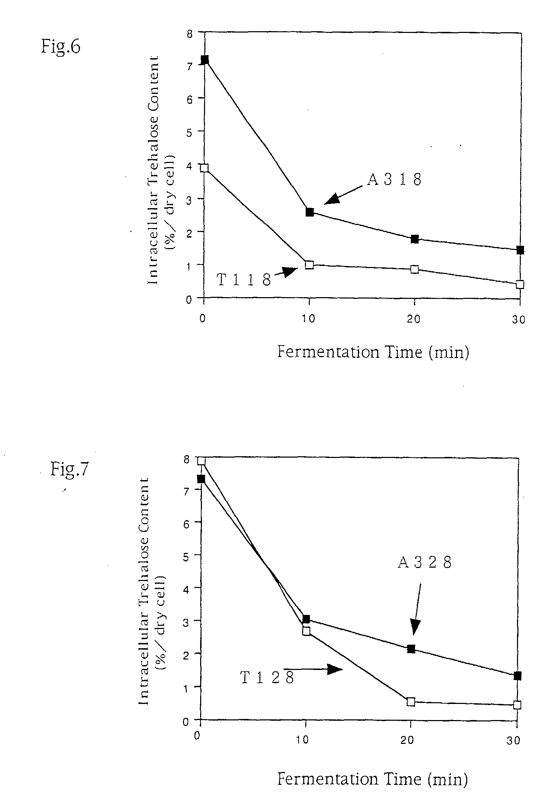

Strains having been cultivated in 30-liter jars each were put into a device for measuring the CO₂ production capacity of the strain in liquid culture, in which the time-dependent variation in the trehalose content of the strain was measured according to the baker's yeast test method of the Yeast Industry Association of Japan. The liquid culture (sucrose 10 %, yeast concentration 2.5 %) was shaken In a liquid culture device for a predetermined period of time. 20 ml of the total amount of the culture was immediately suspended in 200 ml of cold water and then centrifuged to wash the cells, which were again washed with 100 ml of cold water. The finally obtained cells were suspended in 5 ml of cold water, and the trehalose content of those cells was measured. The data obtained are shown in Fig. 6 and Fig. 7.

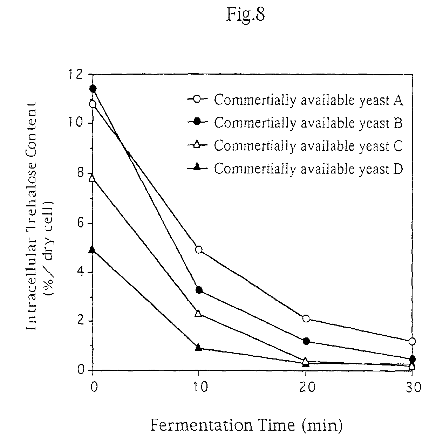

The data of commercially-available yeasts tested in the same manner as above are shown in Fig. 8. Those data verify that the trehalose content retentiveness of each ATH1 gene-disrupted strain was significantly increased. The increase in the trehalose content retention of the gene-disrupted strains is significant, as compared with that in the trehalose content retentiveness of the commercially-available yeasts. The increase was not seen in the laboratory strains, and was specifically seen only in the practical baker's yeast strains. The data obtained herein indicate the time-dependent reduction in the trehalose content of the cells in liquid culture but not in dough. It is believed that the same phenomenon as in the liquid culture occurs also in the incubation of pre-frozen dough. Therefore, it is known that the trehalose content of ATH1 gene-disrupted yeast cells in dough is kept high in the step of pre-freezing the dough. The ATH1 gene-disrupted yeasts of the invention, of which the reduction in the trehalose content was significantly prevented, retained a higher trehalose content during incubation of pre-frozen dough. On the other hand, while high-sugar dough comprising any of the ATH1 gene-disrupted yeasts of the invention is fermented, the intracellular trehalose content of the yeast in the dough is kept high and the intracellular osmotic pressure in the yeast therein is also kept high all the time during the fermentation.

### Example 4:

### (Baking capabilities of yeast strain in dough for loaves and in dough for buns )

Practical baker's yeasts of diploid strains T118 and T128, and their ATH1 gene-disrupted strains A318 and A328, all shown in Table 2, and also a commercially-available baker's yeast (manufactured by Oriental Yeast Industry Co.) were used in preparing dough samples having the composition mentioned below. The samples were tested in accordance with the baker's yeast test method of the Yeast Industry Association of Japan, under the baking condition shown in Table 3.

| | Low-sugar Dough (for loaves) | High-sugar Dough |
|---|---|---|
| (Formulation) | | |
| Wheat flour | 100 | 100 |
| Sugar | 5 | 30 |
| Salt | 2 | 0.5 |
| Yeast | 2 | 4 |
| Shortening | 5 | 6 |
| Skim milk | 0 | 2 |
| Water | 65 | 52 |

**Table 3 -**

| Baking Condition | | |
|---|---|---|
| Mixing | L2,M2,↓ L2,M2,H2∼ | L3,M2,↓ L2,M2,H1∼ |
| Mixing Temperature | 28°C | 28°C |
| First Fermentation | 30°C, 70 min | 30°C, 70 min |
| Second Fermentation | 30°C, 30 min | 30°C, 30 min |
| Dividing, Rounding | 450 g | 450 g |
| Bench | 35°C, 15 min | 35°C, 20 min |
| Shaping | One loaf | One loaf |
| Fermentation in Roaster | 38°C, RH 85 %, 1.5 cm above loaf cup holder | 38°C, RH 85 %, 1.5 cm above loaf cup holder |
| Baking | 200°C, 25 min | 200°C, 25 min |

The data obtained are shown in Table 4 and Table 5 below. As is obvious from the data in Table 4, the wild strains and the ATH1 gene-disrupted strains produced nearly the same results in the loaf dough baking test.

**Table 4 -**

| Loaf Dough Baking Test | | |
|---|---|---|
| Strain Used | Volume of Loaf (ml) | Relative Volume of Loaf (ml/g) |
| T118 | 1,780 | 4.7 |
| A318 | 1,855 | 5.2 |
| T128 | 1,905 | 5.3 |
| A328 | 1,905 | 5.3 |
| Commercially-available Yeast | 1,855 | 5.1 |

**Table 5 -**

| Bun Dough Baking Test | | |
|---|---|---|
| Strain Used | Volume of Bun (ml) | Relative Volume of Bun (ml/g) |
| T118 | 1,275 | 3.1 |
| A318 | 1,810 | 4.7 |
| T128 | 1,510 | 4.0 |
| A328 | 1,740 | 4.3 |
| Commercially-available Yeast | 1,750 | 4.5 |

As is obvious from the data in Table 5, the ATH1 gene-disrupted strains A318 and A328 are significantly superior to the wild strains T118 and T128, respectively, with respect to the baking capability in the bun dough baking test (high-sugar dough resistance). In addition, the baking capability of the strains A318 and A328 is comparable to or better than that of the commercially-available yeast. This supports the high commercial value of the strains A318 and A328.

### Example 5:

### (Gas production of frozen dough)

Practical baker's yeasts of diploid strains T118 (FERM BP-6096) and T128 (FERM BP-6097), and their ATH1 gene-disrupted strains A318 (FERM BP-6039) and A328 (FERM BP-6040), all shown in Table 2 (Mating Matrix), were used in preparing frozen dough samples. The samples were tested in accordance with the baker's yeast test method of the Yeast Industry Association of Japan.

| | Sugarless Dough | Low-sugar Douch (for loaves) | High-sugar Dough |
|---|---|---|---|
| Wheat flour | 100 g | 100 g | 100 g |
| Sugar | 0 g | 5 g | 30 g |
| Salt | 2 g | 2 g | 0.5 g |
| Yeast | 4 g | 4 g | 6 g |
| Water | 65 ml | 65 ml | 52 ml |

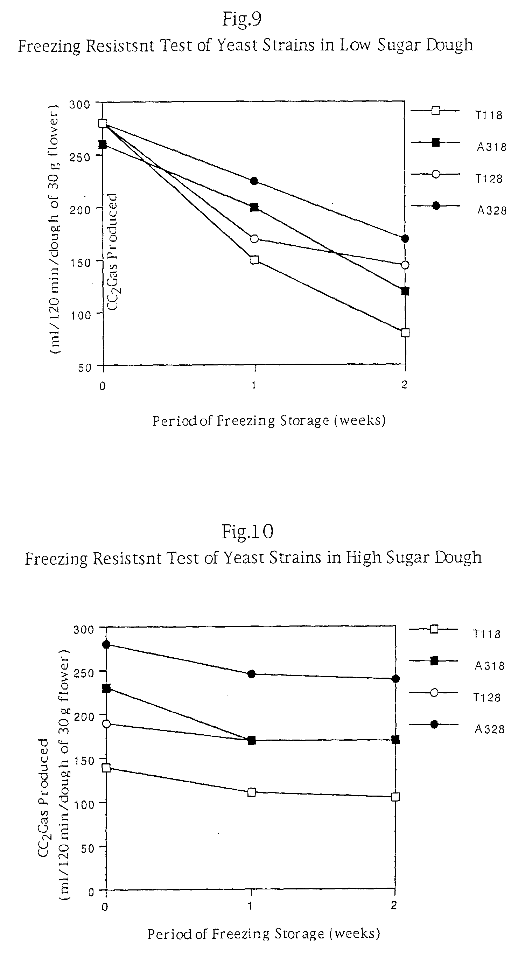

Each yeast was added to the dough having any of the above-mentioned compositions, mixed, and divided into plural portions each having a wheat flour content of 30 g. These were incubated at 30°C for 60 minutes, shaped, then frozen and stored for 1 to 3 weeks, and thereafter thawed, whereupon the gaseous volume of each sample as thawed and kept at 30°C for 120 minutes was measured through fermography. The data obtained are shown in Fig. 9 (low-sugar dough) and Fig. 10 (high-sugar dough). It is known that the ATH1 gene-disrupted strain, A318 exhibited higher freezing resistance in the low-sugar dough samples than the non-disrupted strain, T118, and that the ATH1 gene-disrupted strain, A328 exhibited higher freezing resistance in all dough samples than the non-disrupted strain, T128. Thus, these data verify that the baker's yeast strains were made resistant to freezing through the gene disruption.

In addition, from the data of high-sugar dough samples (Fig. 10), admitted is better freezing resistance of the gene-disrupted strains. This is because of the synergistic effect of the gene-disrupted strains of which both the frozen dough resistance and the high-sugar dough resistance were increased.

### Effects of the Invention:

According to the invention, it is possible to obtain diploid or higher polyploid, practical baker's yeasts with good frozen dough resistance/high-sugar dough resistance, by mating one or more ATH1 gene-disrupted haploid yeasts as produced through gene manipulation of disrupting the ATH1 gene of a haploid yeast, of which the diploid is practical baker's yeast.

The frozen dough-resistant, practical baker's yeasts of the invention has high trehalose-accumulating ability in their culture, and has a reduced ability of trehalose degradation at fermentation process brought by operation before freeze, whereby it possesses trehalose at high concentrate, and the dough comprising the yeast of the invention shows a long term stability of the frozen dough. In addition, since the intracellular osmotic pressure in the yeasts of the invention is high, the yeasts are resistant to high sugar concentration in dough, and are effective for favorably fermenting high-sugar dough.

### Sequence Listing:

Sequence Numbers 1 and 2 indicate primer 1 and primer 2, respectively, for PCR. Sequence Number 3 indicates the amino acid-coding region of ATH1 gene. Sequence Number 4 indicates the full-length region of URA3 gene. Sequence Number 5 indicates the amino acid-coding region of ATH1 gene as cleaved with URA3 gene.

Tables 6 to 25 below show the sequences of Sequence Numbers 1 to 5.

### Annex to the description

Table 6:

Sequence Listing:

Sequence Number: 1

Length of Sequence: 20

Type of Sequence: nucleic acid

Number of Chain: single-stranded

Topology: linear

Kind of Sequence: other nucleic acid, synthetic DNA

Sequence:

5'-CATCCACTGGGAGTGGTTTC

Table 7:

Sequence Number: 2

Length of Sequence: 20

Type of Sequence: nucleic acid

Number of Chain: single-stranded

Topology: linear

Kind of Sequence: other nucleic acid, synthetic DNA

Sequence:

5'-CGAGATGATTGCCAATGTCT

Table 8:

Sequence Number: 3

Length of Sequence: 3126

Type of Sequence: nucleic acid

Topology: linear

Kind of Sequence: genomic DNA

Source:

Name: Saccharomyces cerevisiae

Strain: wild type

Sequence:

Table 9:

ATG ATT TTG GGA AGT AAC TTG TTC TCA AAG AAT ACA TAC TCG AGA 45
Met Ile Leu Gly Ser Asn Leu Phe Ser Lys Asn Thr Tyr Ser Arg
                5                   10                  15

CAA CCA TAT GTT GCT AAC GGT TAT ATA GGT AGT CGT ATT CCC AAT 90
Gln Pro Tyr Val Ala Asn Gly Tyr Ile Gly Ser Arg Ile Pro Asn
                20                  25                  30

ATT GGG TTC GGC TAT GCC TTA GAC ACC CTG AAT TTT TAC ACA GAC 135
Ile Gly Phe Gly Tyr Ala Leu Asp Thr Leu Asn Phe Tyr Thr Asp
                35                  40                  45

GCA CCA GGC GCT TTG AAT AAC GGT TGG CCC TTA AGA AAT CAT AGA 180
Ala Pro Gly Ala Leu Asn Asn Gly Trp Pro Leu Arg Asn His Arg
                50                  55                  60

TTT GCC GGT GCG TTT GTA TCG GAC TTT TAT TGT CTA CAA CCA AAA 225
Phe Ala Gly Ala Phe Val Ser Asp Phe Tyr Cys Leu Gln Pro Lys
                65                  70                  75

CTA AAT TCA ACA AAC TTC CCA GAA TTG GAT GAT GTA GGA TAT TCC 270
Leu Asn Ser Thr Asn Phe Pro Glu Leu Asp Asp Val Gly Tyr Ser
                80                  85                  90

ACT GTC ATT TCA TCT ATT CCA CAA TGG ACC AAT CTA CAG TTC TCA 315
Thr Val Ile Ser Ser Ile Pro Gln Trp Thr Asn Leu Gln Phe Ser
                95                  100                 105

TTA GTG AAT GAT TCT AAG TGG TTC AAT CCA CAA AAT GTT ACG TTG 360
Leu Val Asn Asp Ser Lys Trp Phe Asn Pro Gln Asn Val Thr Leu
                110                 115                 120

GAT GAC GTA ACT AAT TAT AGC CAA AAC TTA TCA ATG AAG GAT GGT 405
Asp Asp Val Thr Asn Tyr Ser Gln Asn Leu Ser Met Lys Asp Gly
                125                 130                 135

Table 10:

ATC GTA ACT ACG GAG TTA GAT TGG CTA AAC AGT CAA ATA CAT GTT    450
Ile Val Thr Thr Glu Leu Asp Trp Leu Asn Ser Gln Ile His Val
                140                 145                 150

AAA AGT GAA ATC TGG GCA CAT CGG CAC ATT CAT CCA CTG GGA GTG    495
Lys Ser Glu Ile Trp Ala His Arg His Ile His Pro Leu Gly Val
                155                 160                 165

GTT TCT TTG GAA ATT TCC CTG AAT ACG GAC CAT TTA CCT TCG GAT    540
Val Ser Leu Glu Ile Ser Leu Asn Thr Asp His Leu Pro Ser Asp
                170                 175                 180

TTT GAT TCA TTA GAT GTT AAT ATA TGG GAT ATA CTT GAT TTC AAC    585
Phe Asp Ser Leu Asp Val Asn Ile Trp Asp Ile Leu Asp Phe Asn
                185                 190                 195

ACA TCA CAT AGG ACT GTT CTA CAT AGC ACG GGA ACA GAC GAA AAA    630
Thr Ser His Arg Thr Val Leu His Ser Thr Gly Thr Asp Glu Lys
                200                 205                 210

AAT AAT GCG GTT TTC ATG ATT GTT CAG CCA GAT AAC GTT CCA TCT    675
Asn Asn Ala Val Phe Met Ile Val Gln Pro Asp Asn Val Pro Ser
                215                 220                 225

TCT AAT TGC GCT ATT TAC TCA ACG TGT ACT GTA AAG TAT GAA AAT    720
Ser Asn Cys Ala Ile Tyr Ser Thr Cys Thr Val Lys Tyr Glu Asn
                230                 235                 240

TCC ACC AAT CCA ATA AAT TCT AGT GAA TCT TTT GAA GAA AAA GAT    765
Ser Thr Asn Pro Ile Asn Ser Ser Glu Ser Phe Glu Glu Lys Asp
                245                 250                 255

GTT TCT TCT AAT ATT TAT AAT GTT ATT TTG ACA GAG GAC CAA CCC    810
Val Ser Ser Asn Ile Tyr Asn Val Ile Leu Thr Glu Asp Gln Pro
                260                 265                 270

Table 11:

AAG ATA ATC GTT CAT AAG TAT GTT GGT ATT ATG TCT ACT GAG TTC 855
Lys Ile Ile Val His Lys Tyr Val Gly Ile Met Ser Thr Glu Phe
275 280 285

AAT AAG AAC AAA GAA CAA CAA GAC AAT ACT AAT ATT GGT TTG GCC 900
Asn Lys Asn Lys Glu Gln Gln Asp Asn Thr Asn Ile Gly Leu Ala
290 295 300

AAA ATG ATA GCT CTA AAC AGT AAA GGC AAT TAC GAG AAG CTT CTG 945
Lys Met Ile Ala Leu Asn Ser Lys Gly Asn Tyr Glu Lys Leu Leu
305 310 315

TCA AGT CAC AAA CGT GCG TGG TAT GAC CTT TAC AAC GAC GCC TTC 990
Ser Ser His Lys Arg Ala Trp Tyr Asp Leu Tyr Asn Asp Ala Phe
320 325 330

ATT GAA ATT CCT TCT GAC AGT CTT TTA GAA ATG ACA GCA AGA TCG 1035
Ile Glu Ile Pro Ser Asp Ser Leu Leu Glu Met Thr Ala Arg Ser
335 340 345

TCC CTA TTC CAT TTA CTA GCA AAT ACA AGA GAT TAC AAT GTC TCG 1080
Ser Leu Phe His Leu Leu Ala Asn Thr Arg Asp Tyr Asn Val Ser
350 355 360

AGC GAT AGG GGT CTT CCC GTG GGA GTT TCT GGT TTG TCA TCA GAT 1125
Ser Asp Arg Gly Leu Pro Val Gly Val Ser Gly Leu Ser Ser Asp
365 370 375

TCC TAT GGT GGT ATG GTG TTC TGG GAC GCA GAT ATA TGG ATG GAA 1170
Ser Tyr Gly Gly Met Val Phe Trp Asp Ala Asp Ile Trp Met Glu
380 385 390

CCT GCC CTA TTG CCT TTC TTC CCA AAT GTG GCT CAA AAT ATG AAT 1215
Pro Ala Leu Leu Pro Phe Phe Pro Asn Val Ala Gln Asn Met Asn
395 400 405

AAT TAC AGA AAT GCT ACA CAT TCG CAG GCA AAG TTA AAT GCA GAG 1260

Asn Tyr Arg Asn Ala Thr His Ser Gln Ala Lys Leu Asn Ala Glu
410 415 420

AAA TAT GGA TAC CCC GGA GCA ATA TAC CCC TGG ACA TCT GGT AAG 1305
Lys Tyr Gly Tyr Pro Gly Ala Ile Tyr Pro Trp Thr Ser Gly Lys
425 430 435

TAC GCT AAT TGT ACT TCT ACG GGA CCT TGT GTC GAT TAC GAA TAC 1350
Tyr Ala Asn Cys Thr Ser Thr Gly Pro Cys Val Asp Tyr Glu Tyr
440 445 450

CAT ATT AAC GTT GAT GTC GCT ATG GCC TCC TTT TCC ATA TAC TTG 1395
His Ile Asn Val Asp Val Ala Met Ala Ser Phe Ser Ile Tyr Leu
455 460 465

AAT GGA CAC GAA GGG ATT GAT GAC GAG TAT CTG AGA TAT ACT ACA 1440
Asn Gly His Glu Gly Ile Asp Asp Glu Tyr Leu Arg Tyr Thr Thr
470 475 480

TGG CCA ATT ATC AAA AAC GCA GCC CAA TTT TTT ACT GCT TAT GTT 1485
Trp Pro Ile Ile Lys Asn Ala Ala Gln Phe Phe Thr Ala Tyr Val
485 490 495

AAG TAC AAT TCT TCC CTA GGA TTG TAT GAA ACA TAT AAT TTG ACA 1530
Lys Tyr Asn Ser Ser Leu Gly Leu Tyr Glu Thr Tyr Asn Leu Thr
500 505 510

GAT CCC GAC GAG TTT GCT AAT CAC ATC AAT AAC GGG GCT TTC ACG 1575
Asp Pro Asp Glu Phe Ala Asn His Ile Asn Asn Gly Ala Phe Thr
515 520 525

AAT GCT GGC ATT AAA ACA CTT CTA AAG TGG GCA ACA GAC ATT GGC 1620
Asn Ala Gly Ile Lys Thr Leu Leu Lys Trp Ala Thr Asp Ile Gly
530 535 540

AAT CAT CTC GGC GAG GTC GTT GAC CCC AAA TGG AGT GAA ATT TCC 1665
Asn His Leu Gly Glu Val Val Asp Pro Lys Trp Ser Glu Ile Ser
545 550 555

Table 12:

AAA GAT ATT TAT ATC CCT AGA TCC TCA TCT AAC ATC ACT TTG GAA 1710
Lys Asp Ile Tyr Ile Pro Arg Ser Ser Ser Asn Ile Thr Leu Glu
560 565 570

TAT TCT GGT ATG AAT AGC TCA GTG GAG ATT AAA CAG GCG GAT GTG 1755
Tyr Ser Gly Met Asn Ser Ser Val Glu Ile Lys Gln Ala Asp Val
575 580 585

ACT TTA ATG GTT TAC CCA CTT GGA TAT ATC AAT GAT GAA TCC ATA 1800
Thr Leu Met Val Tyr Pro Leu Gly Tyr Ile Asn Asp Glu Ser Ile
590 595 600

TTG AAC AAT GCA ATT AAA GAT CTT TAT TAT TAT TCA GAA AGA CAG 1845
Leu Asn Asn Ala Ile Lys Asp Leu Tyr Tyr Tyr Ser Glu Arg Gln
605 610 615

TCT GCG TCT GGG CCT GCA ATG ACA TAT CCG GTT TTT GTG GCC GCA 1890
Ser Ala Ser Gly Pro Ala Met Thr Tyr Pro Val Phe Val Ala Ala
620 625 630

GCT GCT GGT CTG CTG AAT CAC GGC TCT TCT TCT CAA AGT TAC TTA 1935
Ala Ala Gly Leu Leu Asn His Gly Ser Ser Ser Gln Ser Tyr Leu
635 640 645

TAT AAA TCG GTT CTT CCA TAC TTA CGG GCT CCT TTC GCT CAA TTT 1980
Tyr Lys Ser Val Leu Pro Tyr Leu Arg Ala Pro Phe Ala Gln Phe
650 655 660

AGT GAG CAA TCA GAC GAC AAC TTT TTA ACA AAC GGA TTA ACC CAG 2025
Ser Glu Gln Ser Asp Asp Asn Phe Leu Thr Asn Gly Leu Thr Gln
665 670 675

CCA GCA TTC CCC TTT TTA ACA GCT AAC GGT GGA TTT CTA CAG AGC 2070
Pro Ala Phe Pro Phe Leu Thr Ala Asn Gly Gly Phe Leu Gln Ser
680 685 690

Table 13:

ATT CTG TTT GGG TTA ACA GGA ATC CGA TAT TCT TAT GAG GTT GAT 2115
Ile Leu Phe Gly Leu Thr Gly Ile Arg Tyr Ser Tyr Glu Val Asp
695 700 705

CCA GAT ACT AAA AAA ATT AAC CGT TTG TTA AGG TTC AAT CCA ATA 2160
Pro Asp Thr Lys Lys Ile Asn Arg Leu Leu Arg Phe Asn Pro Ile
710 715 720

GAA CTA CCG TTG CTC CCT GGT GGT ATC GCT ATT AGA AAC TTC AAA 2205
Glu Leu Pro Leu Leu Pro Gly Gly Ile Ala Ile Arg Asn Phe Lys
725 730 735

TAT ATG AAC CAA GTT TTA GAT ATA ATA ATT GAC GAC CAC AAT GGT 2250
Tyr Met Asn Gln Val Leu Asp Ile Ile Ile Asp Asp His Asn Gly
740 745 750

ACG ATT GTT CAT AAA TCA GGA GAT GTT CCT ATT CAT ATA AAG ATA 2295
Thr Ile Val His Lys Ser Gly Asp Val Pro Ile His Ile Lys Ile
755 760 765

CCA AAC AGA TCT CTA ATA CAT GAC CAG GAT ATC AAC TTC TAT AAT 2340
Pro Asn Arg Ser Leu Ile His Asp Gln Asp Ile Asn Phe Tyr Asn
770 775 780

GGT TCC GAA AAC GAA AGA AAA CCA AAT CTA GAG CGT AGA GAC GTC 2385
Gly Ser Glu Asn Glu Arg Lys Pro Asn Leu Glu Arg Arg Asp Val
785 790 795

GAC CGT GTT GGT GAT CCA ATG AGG ATG GAT AGG TAT GGT ACC TAT 2430
Asp Arg Val Gly Asp Pro Met Arg Met Asp Arg Tyr Gly Thr Tyr
800 805 810

TAT CTT TTA AAA CCG AAA CAA GAG CTT ACA GTC CAA CTG TTC AAG 2475
Tyr Leu Leu Lys Pro Lys Gln Glu Leu Thr Val Gln Leu Phe Lys
815 820 825

Table 14:

CCT GGC TTA AAC GCA AGA AAC AAC ATA GCG GAA AAT AAG CAA ATA   2520
Pro Gly Leu Asn Ala Arg Asn Asn Ile Ala Glu Asn Lys Gln Ile
                830                 835                 840

ACA AAC TTG ACG GCC GGC GTT CCT GGT GAC GTT GCA TTC TCT GCT   2565
Thr Asn Leu Thr Ala Gly Val Pro Gly Asp Val Ala Phe Ser Ala
                845                 850                 855

CTA GAT GGG AAT AAT TAC ACG CAT TGG CAA CCC TTA GAC AAA ATT   2610
Leu Asp Gly Asn Asn Tyr Thr His Trp Gln Pro Leu Asp Lys Ile
                860                 865                 870

CAC CGT GCG AAG CTA TTG ATT GAT TTA GGT GAA TAC AAC GAG AAA   2655
His Arg Ala Lys Leu Leu Ile Asp Leu Gly Glu Tyr Asn Glu Lys
                875                 880                 885

GAG ATT ACC AAG GGA ATG ATT CTT TGG GGG CAG AGG CCC GCA AAA   2700
Glu Ile Thr Lys Gly Met Ile Leu Trp Gly Gln Arg Pro Ala Lys
                890                 895                 900

AAC ATT TCC ATA TCT ATT TTG CCT CAT TCT GAA AAA GTC GAA AAT   2745
Asn Ile Ser Ile Ser Ile Leu Pro His Ser Glu Lys Val Glu Asn
                905                 910                 915

TTA TTT GCG AAC GTG ACA GAA ATT ATG CAA AAT TCG GGA AAT GAT   2790
Leu Phe Ala Asn Val Thr Glu Ile Met Gln Asn Ser Gly Asn Asp
                920                 925                 930

CAA CTT CTT AAT GAA ACC ATT GGT CAG CTT TTA GAT AAT GCC GGA   2835
Gln Leu Leu Asn Glu Thr Ile Gly Gln Leu Leu Asp Asn Ala Gly
                935                 940                 945

ATT CCT GTC GAG AAC GTT ATT GAT TTT GAT GGC ATA GAA CAA GAG   2880
Ile Pro Val Glu Asn Val Ile Asp Phe Asp Gly Ile Glu Gln Glu
                950                 955                 960

Table 15:

GAT GAT GAA TCT TTG GAT GAT GTG CAA GCC TTA TTG CAC TGG AAG 2925
Asp Asp Glu Ser Leu Asp Asp Val Gln Ala Leu Leu His Trp Lys
965 970 975

AAG GAA GAC TTA GCC AAG CTA ATT GAA CAA ATA CCC AGA CTT AAC 2970
Lys Glu Asp Leu Ala Lys Leu Ile Glu Gln Ile Pro Arg Leu Asn
980 985 990

TTT CTA AAA AGA AAA TTT GTG AAA ATT CTG GAT AAC GTG CCA GTG 3015
Phe Leu Lys Arg Lys Phe Val Lys Ile Leu Asp Asn Val Pro Val
995 1000 1005

AGC CCA AGT GAG CCA TAC TAC GAA GCA AGT CGC AAC CAG TCG TTA 3060
Ser Pro Ser Glu Pro Tyr Tyr Glu Ala Ser Arg Asn Gln Ser Leu
1010 1015 1020

ATC GAG ATA TTA CCC AGT AAT AGA ACG ACT TTC ACT ATT GAT TAT 3105
Ile Glu Ile Leu Pro Ser Asn Arg Thr Thr Phe Thr Ile Asp Tyr
1025 1030 1035

GAT AAA TTT GCA GGT GGG TGA 3126
Asp Lys Phe Ala Gly Gly ***
1041

Table 16:

Sequence Number: 4

Length of Sequence: 804

Type of Sequence: nucleic acid

Topology: linear

Kind of Sequence: genomic DNA

Source:

Name: Saccharomyces cerevisiae

Strain: wild type

Sequence:

Table 17:

ATG TCG AAA GCT ACA TAT AAG GAA CGT GCT GCT ACT CAT CCT AGT 45
Met Ser Lys Ala Thr Tyr Lys Glu Arg Ala Ala Thr His Pro Ser
                5                   10                  15

CCT GTT GCT GCC AAG CTA TTT AAT ATC ATG CAC GAA AAG CAA ACA 90
Pro Val Ala Ala Lys Leu Phe Asn Ile Met His Glu Lys Gln Thr
                20                  25                  30

AAC TTG TGT GCT TCA TTG GAT GTT CGT ACC ACC AAG GAA TTA CTG 135
Asn Leu Cys Ala Ser Leu Asp Val Arg Thr Thr Lys Glu Leu Leu
                35                  40                  45

GAG TTA GTT GAA GCA TTA GGT CCC AAA ATT TGT TTA CTA AAA ACA 180
Glu Leu Val Glu Ala Leu Gly Pro Lys Ile Cys Leu Leu Lys Thr
                50                  55                  60

CAT GTG GAT ATC TTG ACT GAT TTT TCC ATG GAG GGC ACA GTT AAG 225
His Val Asp Ile Leu Thr Asp Phe Ser Met Glu Gly Thr Val Lys
                65                  70                  75

CCG CTA AAG GCA TTA TCC GCC AAG TAC AAT TTT TTA CTC TTC GAA 270
Pro Leu Lys Ala Leu Ser Ala Lys Tyr Asn Phe Leu Leu Phe Glu
                80                  85                  90

GAC AGA AAA TTT GCT GAC ATT GGT AAT ACA GTC AAA TTG CAG TAC 315
Asp Arg Lys Phe Ala Asp Ile Gly Asn Thr Val Lys Leu Gln Tyr
                95                  100                 105

TCT GCG GGT GTA TAC AGA ATA GCA GAA TGG GCA GAC ATT ACG AAT 360
Ser Ala Gly Val Tyr Arg Ile Ala Glu Trp Ala Asp Ile Thr Asn
                110                 115                 120

GCA CAC GGT GTG GTG GGC CCA GGT ATT GTT AGC GGT TTG AAG CAG 405
Ala His Gly Val Val Gly Pro Gly Ile Val Ser Gly Leu Lys Gln
                125                 130                 135

Table 18:

GCG GCA GAA GAA GTA ACA AAG GAA CCT AGA GGC CTT TTG ATG TTA 450
Ala Ala Glu Glu Val Thr Lys Glu Pro Arg Gly Leu Leu Met Leu
140 145 160

GCA GAA TTG TCA TGC AAG GGC TCC CTA TCT ACT GGA GAA TAT ACT 495
Ala Glu Leu Ser Cys Lys Gly Ser Leu Ser Thr Gly Glu Tyr Thr
165 170 175

AAG GGT ACT GTT GAC ATT GCG AAG AGC GAC AAA GAT TTT GTT ATC 540
Lys Gly Thr Val Asp Ile Ala Lys Ser Asp Lys Asp Phe Val Ile
180 185 190

GGC TTT ATT GCT CAA AGA GAC ATG GGT GGA AGA GAT GAA GGT TAC 585
Gly Phe Ile Ala Gln Arg Asp Met Gly Gly Arg Asp Glu Gly Tyr
195 200 205

GAT TGG TTG ATT ATG ACA CCC GGT GTG GGT TTA GAT GAC AAG GGA 630
Asp Trp Leu Ile Met Thr Pro Gly Val Gly Leu Asp Asp Lys Gly
210 215 220

GAC GCA TTG GGT CAA CAG TAT AGA ACC GTG GAT GAT GTG GTC TCT 675
Asp Ala Leu Gly Gln Gln Tyr Arg Thr Val Asp Asp Val Val Ser
225 230 235

ACA GGA TCT GAC ATT ATT ATT GTT GGA AGA GGA CTA TTT GCA AAG 720
Thr Gly Ser Asp Ile Ile Ile Val Gly Arg Gly Leu Phe Ala Lys
240 245 250

GGA AGG GAT GCT AAG GTA GAG GGT GAA CGT TAC AGA AAA GCA GGC 765
Gly Arg Asp Ala Lys Val Glu Gly Glu Arg Tyr Arg Lys Ala Gly
255 260 265

TGG GAA GCA TAT TTG AGA AGA TGC GGC CAG CAA AAC TAA 804
Trp Glu Ala Tyr Leu Arg Arg Cys Gly Gln Gln Asn ***>
270 275 277

Table 19:

Sequence Number: 5

Length of Sequence: 4292

Type of Sequence: nucleic acid

Topology: linear

Kind of Sequence: genomic DNA

Source:

Name: Saccharomyces cerevisiae

Strain: wild type

Sequence:

Table 20:

10 20 30 40 50 60
* * * * * *
ATGATTTTGG GAAGTAACTT GTTCTCAAAG AATACATACT CGAGACAACC ATATGTTGCT

70 80 90 100 110 120
* * * * * *
AACGGTTATA TAGGTAGTCG TATTCCCAAT ATTGGGTTCG GCTATGCCTT AGACACCCTG

130 140 150 160 170 180
* * * * * *
AATTTTTACA CAGACGCACC AGGCGCTTTG AATAACGGTT GGCCCTTAAG AAATCATAGA

190 200 210 220 230 240
* * * * * *
TTTGCCGGTG CGTTTGTATC GGACTTTTAT TGTCTACAAC CAAAACTAAA TTCAACAAAC

250 260 270 280 290 300
* * * * * *
TTCCCAGAAT TGGATGATGT AGGATATTCC ACTGTCATTT CATCTATTCC ACAATGGACC

310 320 330 340 350 360
* * * * * *
AATCTACAGT TCTCATTAGT GAATGATTCT AAGTGGTTCA ATCCACAAAA TGTTACGTTG

370 380 390 400 410 420
* * * * * *
GATGACGTAA CTAATTATAG CCAAAACTTA TCAATGAAGG ATGGTATCGT AACTACGGAG

430 440 450 460 470 480
* * * * * *
TTAGATTGGC TAAACAGTCA AATACATGTT AAAAGTGAAA TCTGGGCACA TCGGCACATT

490 500 510 520 530 540
* * * * * *
CATCCACTGG GAGTGGTTTC TTTGGAAATT TCCCTGAATA CGGACCATTT ACCTTCGGAT

550 560 570 580 590 600
* * * * * *
TTTGATTCAT TAGATGTTAA TATATGGGAT ATACTTGATT TCAACACATC ACATAGGACT

610 620 630 640 650 660
* * * * * *
GTTCTACATA GCACGGGAAC AGACGAAAAA AATAATGCGG TTTTCATGAT TGTTCAGCCA

Table 21:

670 680 690 700 710 720
* * * * * *
GATAACGTTC CATCTTCTAA TTGCGCTATT TACTCAACGT GTACTGTAAA GTATGAAAAT

730 740 750 760 770 780
* * * * * *
TCCACCAATC CAATAAATTC TAGTGAATCT TTTGAAGAAA AAGATGTTTC TTCTAATATT

790 800 810 820 830 840
* * * * * *
TATAATGTTA TTTTGACAGA GGACCAACCC AAGATAATCG TTCATAAGTA TGTTGGTATT

850 860 870 880 890 900
* * * * * *
ATGTCTACTG AGTTCAATAA GAACAAAGAA CAACAAGACA ATACTAATAT TGGTTTGGCC

910 920 930 940 950 960
* * * * * *
AAAATGATAG CTCTAAACAG TAAAGGCAAT TACGAGAAGC TTTTCAATTC AATTCATCAT

970 980 990 1000 1010 1020
* * * * * *
TTTTTTTTTA TTCTTTTTTT TGATTTCGGT TTCTTTGAAA TTTTTTTGAT TCGGTAATCT

1030 1040 1050 1060 1070 1080
* * * * * *
CCGAACAGAA GGAAGAACGA AGGAAGGAGC ACAGACTTAG ATTGGTATAT ATACGCATAT

1090 1100 1110 1120 1130 1140
* * * * * *
GTAGTGTTGA AGAAACATGA AATTGCCCAG TATTCTTAAC CCAACTGCAC AGAACAAAAA

1150 1160 1170 1180 1190 1200
* * * * * *
CCTGCAGGAA ACGAAGATAA ATCATGTCGA AAGCTACATA TAAGGAACGT GCTGCTACTC

1210 1220 1230 1240 1250 1260
* * * * * *
ATCCTAGTCC TGTTGCTGCC AAGCTATTTA ATATCATGCA CGAAAAGCAA ACAAACTTGT

1270 1280 1290 1300 1310 1320
* * * * * *
GTGCTTCATT GGATGTTCGT ACCACCAAGG AATTACTGGA GTTAGTTGAA GCATTAGGTC

Table 22:

1330 1340 1350 1360 1370 1380
* * * * * *
CCAAAATTTG TTTACTAAAA ACACATGTGG ATATCTTGAC TGATTTTTCC ATGGAGGGCA

1390 1400 1410 1420 1430 1440
* * * * * *
CAGTTAAGCC GCTAAAGGCA TTATCCGCCA AGTACAATTT TTTACTCTTC GAAGACAGAA

1450 1460 1470 1480 1490 1500
* * * * * *
AATTTGCTGA CATTGGTAAT ACAGTCAAAT TGCAGTACTC TGCGGGTGTA TACAGAATAG

1510 1520 1530 1540 1550 1560
* * * * * *
CAGAATGGGC AGACATTACG AATGCACACG GTGTGGTGGG CCCAGGTATT GTTAGCGGTT

1570 1580 1590 1600 1610 1620
* * * * * *
TGAAGCAGGC GGCAGAAGAA GTAACAAAGG AACCTAGAGG CCTTTTGATG TTAGCAGAAT

1630 1640 1650 1660 1670 1680
* * * * * *
TGTCATGCAA GGGCTCCCTA TCTACTGGAG AATATACTAA GGGTACTGTT GACATTGCGA

1690 1700 1710 1720 1730 1740
* * * * * *
AGAGCGACAA AGATTTTGTT ATCGGCTTTA TTGCTCAAAG AGACATGGGT GGAAGAGATG

1750 1760 1770 1780 1790 1800
* * * * * *
AAGGTTACGA TTGGTTGATT ATGACACCCG GTGTGGGTTT AGATGACAAG GGAGACGCAT

1810 1820 1830 1840 1850 1860
* * * * * *
TGGGTCAACA GTATAGAACC GTGGATGATG TGGTCTCTAC AGGATCTGAC ATTATTATTG

1870 1880 1890 1900 1910 1920
* * * * * *
TTGGAAGAGG ACTATTTGCA AAGGGAAGGG ATGCTAAGGT AGAGGGTGAA CGTTACAGAA

1930 1940 1950 1960 1970 1980
* * * * * *
AAGCAGGCTG GGAAGCATAT TTGAGAAGAT GCGGCCAGCA AAACTAAAAA ACTGTATTAT

Table 23:

1990 2000 2010 2020 2030 2040
* * * * * *
AAGTAAATGC ATGTATACTA AACTCACAAA TTAGAGCTTC AATTTAATTA TATCAGTTAT

2050 2060 2070 2080 2090 2100
* * * * * *
TACCCGGGAA TCTCGGTCGT AATGATTTTT ATAATGACGA AAAAAAAAAA ATTGGAAAGA

2110 2120 2130 2140 2150 2160
* * * * * *
AAAAGCTTCT GTCAAGTCAC AAACGTGCGT GGTATGACCT TTACAACGAC GCCTTCATTG

2170 2180 2190 2200 2210 2220
* * * * * *
AAATTCCTTC TGACAGTCTT TTAGAAATGA CAGCAAGATC GTCCCTATTC CATTTACTAG

2230 2240 2250 2260 2270 2280
* * * * * *
CAAATACAAG AGATTACAAT GTCTCGAGCG ATAGGGGTCT TCCCGTGGGA GTTTCTGGTT

2290 2300 2310 2320 2330 2340
* * * * * *
TGTCATCAGA TTCCTATGGT GGTATGGTGT TCTGGGACGC AGATATATGG ATGGAACCTG

2350 2360 2370 2380 2390 2400
* * * * * *
CCCTATTGCC TTTCTTCCCA AATGTGGCTC AAAATATGAA TAATTACAGA AATGCTACAC

2410 2420 2430 2440 2450 2460
* * * * * *
ATTCGCAGGC AAAGTTAAAT GCAGAGAAAT ATGGATACCC CGGAGCAATA TACCCCTGGA

2470 2480 2490 2500 2510 2520
* * * * * *
CATCTGGTAA GTACGCTAAT TGTACTTCTA CGGGACCTTG TGTCGATTAC GAATACCATA

2530 2540 2550 2560 2570 2580
* * * * * *
TTAACGTTGA TGTCGCTATG GCCTCCTTTT CCATATACTT GAATGGACAC GAAGGGATTG

2590 2600 2610 2620 2630 2640
* * * * * *
ATGACGAGTA TCTGAGATAT ACTACATGGC CAATTATCAA AAACGCAGCC CAATTTTTTA

2650 2660 2670 2680 2690 2700
* * * * * *
CTGCTTATGT TAAGTACAAT TCTTCCCTAG GATTGTATGA AACATATAAT TTGACAGATC

Table 24:

2710 2720 2730 2740 2750 2760
* * * * * *
CCGACGAGTT TGCTAATCAC ATCAATAACG GGGCTTTCAC GAATGCTGGC ATTAAAACAC

2770 2780 2790 2800 2810 2820
* * * * * *
TTCTAAAGTG GGCAACAGAC ATTGGCAATC ATCTCGGCGA GGTCGTTGAC CCCAAATGGA

2830 2840 2850 2860 2870 2880
* * * * * *
GTGAAATTTC CAAAGATATT TATATCCCTA GATCCTCATC TAACATCACT TTGGAATATT

2890 2900 2910 2920 2930 2940
* * * * * *
CTGGTATGAA TAGCTCAGTG GAGATTAAAC AGGCGGATGT GACTTTAATG GTTTACCCAC

2950 2960 2970 2980 2990 3000
* * * * * *
TTGGATATAT CAATGATGAA TCCATATTGA ACAATGCAAT TAAAGATCTT TATTATTATT

3010 3020 3030 3040 3050 3060
* * * * * *
CAGAAAGACA GTCTGCGTCT GGGCCTGCAA TGACATATCC GGTTTTTGTG GCCGCAGCTG

3070 3080 3090 3100 3110 3120
* * * * * *
CTGGTCTGCT GAATCACGGC TCTTCTTCTC AAAGTTACTT ATATAAATCG GTTCTTCCAT

3130 3140 3150 3160 3170 3180
* * * * * *
ACTTACGGGC TCCTTTCGCT CAATTTAGTG AGCAATCAGA CGACAACTTT TTAACAAACG

3190 3200 3210 3220 3230 3240
* * * * * *
GATTAACCCA GCCAGCATTC CCCTTTTTAA CAGCTAACGG TGGATTTCTA CAGAGCATTC

3250 3260 3270 3280 3290 3300
* * * * * *
TGTTTGGGTT AACAGGAATC CGATATTCTT ATGAGGTTGA TCCAGATACT AAAAAAATTA

3310 3320 3330 3340 3350 3360
* * * * * *
ACCGTTTGTT AAGGTTCAAT CCAATAGAAC TACCGTTGCT CCCTGGTGGT ATCGCTATTA

3370 3380 3390 3400 3410 3420
* * * * * *
GAAACTTCAA ATATATGAAC CAAGTTTTAG ATATAATAAT TGACGACCAC AATGGTACGA

Table 25:

3430 3440 3450 3460 3470 3480
* * * * * *
TTGTTCATAA ATCAGGAGAT GTTCCTATTC ATATAAAGAT ACCAAACAGA TCTCTAATAC

3490 3500 3510 3520 3530 3540
* * * * * *
ATGACCAGGA TATCAACTTC TATAATGGTT CCGAAAACGA AAGAAAACCA AATCTAGAGC

3550 3560 3570 3580 3590 3600
* * * * * *
GTAGAGACGT CGACCGTGTT GGTGATCCAA TGAGGATGGA TAGGTATGGT ACCTATTATC

3610 3620 3630 3640 3650 3660
* * * * * *
TTTTAAAACC GAAACAAGAG CTTACAGTCC AACTGTTCAA GCCTGGCTTA AACGCAAGAA

3670 3680 3690 3700 3710 3720
* * * * * *
ACAACATAGC GGAAAATAAG CAAATAACAA ACTTGACGGC CGGCGTTCCT GGTGACGTTG

3730 3740 3750 3760 3770 3780
* * * * * *
CATTCTCTGC TCTAGATGGG AATAATTACA CGCATTGGCA ACCCTTAGAC AAAATTCACC

3790 3800 3810 3820 3830 3840
* * * * * *
GTGCGAAGCT ATTGATTGAT TTAGGTGAAT ACAACGAGAA AGAGATTACC AAGGGAATGA

3850 3860 3870 3880 3890 3900
* * * * * *
TTCTTTGGGG GCAGAGGCCC GCAAAAAACA TTTCCATATC TATTTTGCCT CATTCTGAAA

3910 3920 3930 3940 3950 3960
* * * * * *
AAGTCGAAAA TTTATTTGCG AACGTGACAG AAATTATGCA AAATTCGGGA AATGATCAAC

3970 3980 3990 4000 4010 4020
* * * * * *
TTCTTAATGA AACCATTGGT CAGCTTTTAG ATAATGCCGG AATTCCTGTC GAGAACGTTA

4030 4040 4050 4060 4070 4080
* * * * * *
TTGATTTTGA TGGCATAGAA CAAGAGGATG ATGAATCTTT GGATGATGTG CAAGCCTTAT

4090 4100 4110 4120 4130 4140
* * * * * *
TGCACTGGAA GAAGGAAGAC TTAGCCAAGC TAATTGAACA AATACCCAGA CTTAACTTTC

Table 26:

4150 4160 4170 4180 4190 4200
* * * * * *
TAAAAAGAAA ATTTGTGAAA ATTCTGGATA ACGTGCCAGT GAGCCCAAGT GAGCCATACT

4210 4220 4230 4240 4250 4260
* * * * * *
ACGAAGCAAG TCGCAACCAG TCGTTAATCG AGATATTACC CAGTAATAGA ACGACTTTCA

4270 4280 4290
* * *
CTATTGATTA TGATAAATTT GCAGGTGGGT GA

## Claims

1. An ATH1 gene-disrupted, haploid yeast as produced through gene manipulation of disrupting the ATH1 gene in a haploid yeast of which the diploid is practical baker's yeast.

2. A method for constructing an ATH1 gene-disrupted, haploid yeast through gene manipulation, comprising inserting a marker such as URA3 into the ATH1 gene in a haploid yeast of which the diploid is practical baker's yeast, to thereby disrupt said ATH1 gene.

3. A diploid or higher polyploid, frozen dough-resistant and high-sugar dough-resistant, practical baker's yeast as produced through mating with one or more ATH1 gene-disrupted, haploid yeasts produced through gene manipulation of disrupting the ATH1 gene in a haploid yeast of which the diploid is practical baker's yeast.

4. An a/α-type, diploid, frozen dough-resistant and high-sugar dough-resistant, practical baker's yeast as produced through mating of an ATH1 gene-disrupted, a-type haploid yeast produced through gene manipulation of disrupting the ATH1 gene in an a-type haploid yeast, with an ATH1 gene-disrupted, α-type haploid yeast produced through gene manipulation of disrupting the ATH1 gene in an α-type haploid yeast.

5. An a/α-type, diploid, frozen dough-resistant and high-sugar dough-resistant, practical baker's yeast as produced through mating of an ATH1 gene-disrupted, a-type haploid yeast produced through gene manipulation of disrupting the ATH1 gene in an a-type haploid yeast of which the diploid is practical baker's yeast, with an ATH1 gene-disrupted, α-type haploid yeast produced through gene manipulation of disrupting the ATH1 gene in an α-type haploid yeast of which the diploid is practical baker's yeast.

6. A method for constructing a diploid or higher polyploid, frozen dough-resistant and high-sugar dough-resistant, practical baker's yeast, which comprises inserting a marker such as URA3 into the ATH1 gene in a haploid yeast, of which the diploid is practical baker's yeast, to thereby disrupt said ATH1 gene, followed by mating one or more of the resulting ATH1 gene-disrupted, haploid yeasts.

7. A frozen dough-resistant and high-sugar dough-resistant, practical baker's yeast as produced through mass-culture of an a/α-type, diploid, frozen dough-resistant and high-sugar dough-resistant, practical baker's yeast produced through mating of an ATH1 gene-disrupted, a-type haploid yeast produced through gene manipulation of disrupting the ATH1 gene in an a-type haploid yeast, with an ATH1 gene-disrupted, α-type haploid yeast produced through gene manipulation of disrupting the ATH1 gene in an α-type haploid yeast.

8. Frozen dough-resistant, practical baker's yeast-containing, frozen dough, as produced by preparing dough with a diploid or higher polyploid, frozen dough-resistant, practical baker's yeast that is produced through mating with one or more ATH1 gene-disrupted, haploid yeasts produced through gene manipulation of disrupting the ATH1 gene in a haploid yeast of which the diploid is practical baker's yeast, then incubating it and thereafter freezing it.

9. High-sugar dough-resistant, practical baker's yeast-containing, high-sugar dough, which comprises a diploid or higher polyploid, high-sugar dough-resistant, practical baker's yeast that is produced through mating with one or more ATH1 gene-disrupted, haploid yeasts produced through gene manipulation of disrupting the ATH1 gene in a haploid yeast of which the diploid is practical baker's yeast.

10. Bread from frozen dough, which is produced by preparing dough with a diploid or higher polyploid, frozen dough-resistant, practical baker's yeast that is produced through mating with one or more ATH1 gene-disrupted, haploid yeasts produced through gene manipulation of disrupting the ATH1 gene in a haploid yeast of which the diploid is practical baker's yeast, then incubating the dough, freezing it to give frozen dough-resistant, practical baker's yeast-containing frozen dough, thawing the resulting frozen dough, fermenting it, and finally baking it.

11. Bread as produced by preparing high-sugar dough with a diploid or higher polyploid, high-sugar dough-resistant, practical baker's yeast that is produced through mating with one or more ATH1 gene-disrupted, haploid yeasts produced through gene manipulation of disrupting the ATH1 gene in a haploid yeast of which the diploid is practical baker's yeast, then fermenting the dough, and finally baking it.


SEQUENCE LISTING

(1) GENERAL INFORMATION:

(i) APPLICANT:
(A) NAME: NATIONAL FOOD RESEARCH INSTITUTE MINISTRY OF
AGRICULTURE, FORESTRY AND ...
(B) STREET: 1-2, Kannondai 2-chome,Tsukuba-shi
(C) CITY: Ibaraki-ken
(D) STATE: -
(E) COUNTRY: JAPAN
(F) POSTAL CODE (ZIP): -
(G) TELEPHONE: -
(H) TELEFAX: -
(I) TELEX: -

(A) NAME: ORIENTAL YEAST CO., LTD.
(B) STREET: 6-10, Azusawa 3-chome,Itabashi-ku
(C) CITY: Tokyo
(D) STATE: -
(E) COUNTRY: JAPAN
(F) POSTAL CODE (ZIP): -
(G) TELEPHONE: -
(H) TELEFAX: -
(I) TELEX: -

(ii) TITLE OF INVENTION: FROZEN DOUGH-RESISTANT AND HIGH-SUGAR
DOUGH-RESISTANT, PRACTICAL BAKER'S YEAST

(iii) NUMBER OF SEQUENCES: 5

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(v) CURRENT APPLICATION DATA:
APPLICATION NUMBER: EP 98403046.0
(vi) PRIOR APPLICATION DATA:
(A) APPLICATION NUMBER: JP 9-352016
(B) FILING DATE: 08-DEC-1997


(2) INFORMATION FOR SEQ ID NO: 1:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: nucleic acid
(A) DESCRIPTION: /desc = "synthetic DNA"

(xi) SEQUENCE DESCRIPTION: SEQ ID NO: 1:

CATCCACTGG GAGTGGTTTC 20

(2) INFORMATION FOR SEQ ID NO: 2:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: nucleic acid
(A) DESCRIPTION: /desc = "synthetic DNA"

(xi) SEQUENCE DESCRIPTION: SEQ ID NO: 2:

CGAGATGATT GCCAATGTCT 20

(2) INFORMATION FOR SEQ ID NO: 3:

(i) SEQUENCE CHARACTERISTICS:
(A) LENGTH: 3126 base pairs
(B) TYPE: nucleic acid
(C) STRANDEDNESS: single
(D) TOPOLOGY: linear

(ii) MOLECULE TYPE: DNA (genomic)

(vi) ORIGINAL SOURCE:
(B) STRAIN: Saccharomyces cerevisiae, wild type

(ix) FEATURE:
(A) NAME/KEY: CDS
(B) LOCATION:1..3123

(xi) SEQUENCE DESCRIPTION: SEQ ID NO: 3:

ATG ATT TTG GGA AGT AAC TTG TTC TCA AAG AAT ACA TAC TCG AGA CAA 48
Met Ile Leu Gly Ser Asn Leu Phe Ser Lys Asn Thr Tyr Ser Arg Gln
1               5                   10                  15

CCA TAT GTT GCT AAC GGT TAT ATA GGT AGT CGT ATT CCC AAT ATT GGG 96
Pro Tyr Val Ala Asn Gly Tyr Ile Gly Ser Arg Ile Pro Asn Ile Gly
            20                  25                  30

TTC GGC TAT GCC TTA GAC ACC CTG AAT TTT TAC ACA GAC GCA CCA GGC 144
Phe Gly Tyr Ala Leu Asp Thr Leu Asn Phe Tyr Thr Asp Ala Pro Gly
        35                  40                  45

GCT TTG AAT AAC GGT TGG CCC TTA AGA AAT CAT AGA TTT GCC GGT GCG 192
Ala Leu Asn Asn Gly Trp Pro Leu Arg Asn His Arg Phe Ala Gly Ala
    50                  55                  60

TTT GTA TCG GAC TTT TAT TGT CTA CAA CCA AAA CTA AAT TCA ACA AAC 240
Phe Val Ser Asp Phe Tyr Cys Leu Gln Pro Lys Leu Asn Ser Thr Asn
65                  70                  75                  80

TTC CCA GAA TTG GAT GAT GTA GGA TAT TCC ACT GTC ATT TCA TCT ATT 288
Phe Pro Glu Leu Asp Asp Val Gly Tyr Ser Thr Val Ile Ser Ser Ile

                    85                  90                  95

CCA CAA TGG ACC AAT CTA CAG TTC TCA TTA GTG AAT GAT TCT AAG TGG        336
Pro Gln Trp Thr Asn Leu Gln Phe Ser Leu Val Asn Asp Ser Lys Trp
            100                 105                 110

TTC AAT CCA CAA AAT GTT ACG TTG GAT GAC GTA ACT AAT TAT AGC CAA        384
Phe Asn Pro Gln Asn Val Thr Leu Asp Asp Val Thr Asn Tyr Ser Gln
        115                 120                 125

AAC TTA TCA ATG AAG GAT GGT ATC GTA ACT ACG GAG TTA GAT TGG CTA        432
Asn Leu Ser Met Lys Asp Gly Ile Val Thr Thr Glu Leu Asp Trp Leu
    130                 135                 140

AAC AGT CAA ATA CAT GTT AAA AGT GAA ATC TGG GCA CAT CGG CAC ATT        480
Asn Ser Gln Ile His Val Lys Ser Glu Ile Trp Ala His Arg His Ile
145                 150                 155                 160

CAT CCA CTG GGA GTG GTT TCT TTG GAA ATT TCC CTG AAT ACG GAC CAT        528
His Pro Leu Gly Val Val Ser Leu Glu Ile Ser Leu Asn Thr Asp His
                165                 170                 175

TTA CCT TCG GAT TTT GAT TCA TTA GAT GTT AAT ATA TGG GAT ATA CTT        576
Leu Pro Ser Asp Phe Asp Ser Leu Asp Val Asn Ile Trp Asp Ile Leu
            180                 185                 190

GAT TTC AAC ACA TCA CAT AGG ACT GTT CTA CAT AGC ACG GGA ACA GAC        624
Asp Phe Asn Thr Ser His Arg Thr Val Leu His Ser Thr Gly Thr Asp
        195                 200                 205

GAA AAA AAT AAT GCG GTT TTC ATG ATT GTT CAG CCA GAT AAC GTT CCA        672
Glu Lys Asn Asn Ala Val Phe Met Ile Val Gln Pro Asp Asn Val Pro
    210                 215                 220

TCT TCT AAT TGC GCT ATT TAC TCA ACG TGT ACT GTA AAG TAT GAA AAT        720
Ser Ser Asn Cys Ala Ile Tyr Ser Thr Cys Thr Val Lys Tyr Glu Asn
225                 230                 235                 240

TCC ACC AAT CCA ATA AAT TCT AGT GAA TCT TTT GAA GAA AAA GAT GTT        768
Ser Thr Asn Pro Ile Asn Ser Ser Glu Ser Phe Glu Glu Lys Asp Val
                245                 250                 255

TCT TCT AAT ATT TAT AAT GTT ATT TTG ACA GAG GAC CAA CCC AAG ATA        816
Ser Ser Asn Ile Tyr Asn Val Ile Leu Thr Glu Asp Gln Pro Lys Ile
            260                 265                 270

ATC GTT CAT AAG TAT GTT GGT ATT ATG TCT ACT GAG TTC AAT AAG AAC        864
Ile Val His Lys Tyr Val Gly Ile Met Ser Thr Glu Phe Asn Lys Asn
        275                 280                 285

AAA GAA CAA CAA GAC AAT ACT AAT ATT GGT TTG GCC AAA ATG ATA GCT        912
Lys Glu Gln Gln Asp Asn Thr Asn Ile Gly Leu Ala Lys Met Ile Ala
    290                 295                 300

CTA AAC AGT AAA GGC AAT TAC GAG AAG CTT CTG TCA AGT CAC AAA CGT        960
Leu Asn Ser Lys Gly Asn Tyr Glu Lys Leu Leu Ser Ser His Lys Arg
305                 310                 315                 320

GCG TGG TAT GAC CTT TAC AAC GAC GCC TTC ATT GAA ATT CCT TCT GAC       1008
Ala Trp Tyr Asp Leu Tyr Asn Asp Ala Phe Ile Glu Ile Pro Ser Asp
                325                 330                 335

AGT CTT TTA GAA ATG ACA GCA AGA TCG TCC CTA TTC CAT TTA CTA GCA       1056
Ser Leu Leu Glu Met Thr Ala Arg Ser Ser Leu Phe His Leu Leu Ala

            340                 345                 350

AAT ACA AGA GAT TAC AAT GTC TCG AGC GAT AGG GGT CTT CCC GTG GGA 1104
Asn Thr Arg Asp Tyr Asn Val Ser Ser Asp Arg Gly Leu Pro Val Gly
        355                 360                 365

GTT TCT GGT TTG TCA TCA GAT TCC TAT GGT GGT ATG GTG TTC TGG GAC 1152
Val Ser Gly Leu Ser Ser Asp Ser Tyr Gly Gly Met Val Phe Trp Asp
    370                 375                 380

GCA GAT ATA TGG ATG GAA CCT GCC CTA TTG CCT TTC TTC CCA AAT GTG 1200
Ala Asp Ile Trp Met Glu Pro Ala Leu Leu Pro Phe Phe Pro Asn Val
385                 390                 395                 400

GCT CAA AAT ATG AAT AAT TAC AGA AAT GCT ACA CAT TCG CAG GCA AAG 1248
Ala Gln Asn Met Asn Asn Tyr Arg Asn Ala Thr His Ser Gln Ala Lys
                405                 410                 415

TTA AAT GCA GAG AAA TAT GGA TAC CCC GGA GCA ATA TAC CCC TGG ACA 1296
Leu Asn Ala Glu Lys Tyr Gly Tyr Pro Gly Ala Ile Tyr Pro Trp Thr
            420                 425                 430

TCT GGT AAG TAC GCT AAT TGT ACT TCT ACG GGA CCT TGT GTC GAT TAC 1344
Ser Gly Lys Tyr Ala Asn Cys Thr Ser Thr Gly Pro Cys Val Asp Tyr
        435                 440                 445

GAA TAC CAT ATT AAC GTT GAT GTC GCT ATG GCC TCC TTT TCC ATA TAC 1392
Glu Tyr His Ile Asn Val Asp Val Ala Met Ala Ser Phe Ser Ile Tyr
    450                 455                 460

TTG AAT GGA CAC GAA GGG ATT GAT GAC GAG TAT CTG AGA TAT ACT ACA 1440
Leu Asn Gly His Glu Gly Ile Asp Asp Glu Tyr Leu Arg Tyr Thr Thr
465                 470                 475                 480

TGG CCA ATT ATC AAA AAC GCA GCC CAA TTT TTT ACT GCT TAT GTT AAG 1488
Trp Pro Ile Ile Lys Asn Ala Ala Gln Phe Phe Thr Ala Tyr Val Lys
                485                 490                 495

TAC AAT TCT TCC CTA GGA TTG TAT GAA ACA TAT AAT TTG ACA GAT CCC 1536
Tyr Asn Ser Ser Leu Gly Leu Tyr Glu Thr Tyr Asn Leu Thr Asp Pro
            500                 505                 510

GAC GAG TTT GCT AAT CAC ATC AAT AAC GGG GCT TTC ACG AAT GCT GGC 1584
Asp Glu Phe Ala Asn His Ile Asn Asn Gly Ala Phe Thr Asn Ala Gly
        515                 520                 525

ATT AAA ACA CTT CTA AAG TGG GCA ACA GAC ATT GGC AAT CAT CTC GGC 1632
Ile Lys Thr Leu Leu Lys Trp Ala Thr Asp Ile Gly Asn His Leu Gly
    530                 535                 540

GAG GTC GTT GAC CCC AAA TGG AGT GAA ATT TCC AAA GAT ATT TAT ATC 1680
Glu Val Val Asp Pro Lys Trp Ser Glu Ile Ser Lys Asp Ile Tyr Ile
545                 550                 555                 560

CCT AGA TCC TCA TCT AAC ATC ACT TTG GAA TAT TCT GGT ATG AAT AGC 1728
Pro Arg Ser Ser Ser Asn Ile Thr Leu Glu Tyr Ser Gly Met Asn Ser
                565                 570                 575

TCA GTG GAG ATT AAA CAG GCG GAT GTG ACT TTA ATG GTT TAC CCA CTT 1776
Ser Val Glu Ile Lys Gln Ala Asp Val Thr Leu Met Val Tyr Pro Leu
            580                 585                 590

GGA TAT ATC AAT GAT GAA TCC ATA TTG AAC AAT GCA ATT AAA GAT CTT 1824
Gly Tyr Ile Asn Asp Glu Ser Ile Leu Asn Asn Ala Ile Lys Asp Leu

        595                 600                 605

TAT TAT TAT TCA GAA AGA CAG TCT GCG TCT GGG CCT GCA ATG ACA TAT        1872
Tyr Tyr Tyr Ser Glu Arg Gln Ser Ala Ser Gly Pro Ala Met Thr Tyr
    610                 615                 620

CCG GTT TTT GTG GCC GCA GCT GCT GGT CTG CTG AAT CAC GGC TCT TCT        1920
Pro Val Phe Val Ala Ala Ala Ala Gly Leu Leu Asn His Gly Ser Ser
625                 630                 635                 640

TCT CAA AGT TAC TTA TAT AAA TCG GTT CTT CCA TAC TTA CGG GCT CCT        1968
Ser Gln Ser Tyr Leu Tyr Lys Ser Val Leu Pro Tyr Leu Arg Ala Pro
                645                 650                 655

TTC GCT CAA TTT AGT GAG CAA TCA GAC GAC AAC TTT TTA ACA AAC GGA        2016
Phe Ala Gln Phe Ser Glu Gln Ser Asp Asp Asn Phe Leu Thr Asn Gly
            660                 665                 670

TTA ACC CAG CCA GCA TTC CCC TTT TTA ACA GCT AAC GGT GGA TTT CTA        2064
Leu Thr Gln Pro Ala Phe Pro Phe Leu Thr Ala Asn Gly Gly Phe Leu
        675                 680                 685

CAG AGC AAT CTG TTT GGG TTA ACA GGA ATC CGA TAT TCT TAT GAG GTT        2112
Gln Ser Asn Leu Phe Gly Leu Thr Gly Ile Arg Tyr Ser Tyr Glu Val
    690                 695                 700

GAT CCA GAT ACT AAA AAA ATT AAC CGT TTG TTA AGG TTC AAT CCA ATA        2160
Asp Pro Asp Thr Lys Lys Ile Asn Arg Leu Leu Arg Phe Asn Pro Ile
705                 710                 715                 720

GAA CTA CCG TTG CTC CCT GGT GGT ATC GCT ATT AGA AAC TTC AAA TAT        2208
Glu Leu Pro Leu Leu Pro Gly Gly Ile Ala Ile Arg Asn Phe Lys Tyr
                725                 730                 735

ATG AAC CAA GTT TTA GAT ATA ATA ATT GAC GAC CAC AAT GGT ACG ATT        2256
Met Asn Gln Val Leu Asp Ile Ile Ile Asp Asp His Asn Gly Thr Ile
            740                 745                 750

GTT CAT AAA TCA GGA GAT GTT CCT ATT CAT ATA AAG ATA CCA AAC AGA        2304
Val His Lys Ser Gly Asp Val Pro Ile His Ile Lys Ile Pro Asn Arg
        755                 760                 765

TCT CTA ATA CAT GAC CAG GAT ATC AAC TTC TAT AAT GGT TCC GAA AAC        2352
Ser Leu Ile His Asp Gln Asp Ile Asn Phe Tyr Asn Gly Ser Glu Asn
    770                 775                 780

GAA AGA AAA CCA AAT CTA GAG CGT AGA GAC GTC GAC CGT GTT GGT GAT        2400
Glu Arg Lys Pro Asn Leu Glu Arg Arg Asp Val Asp Arg Val Gly Asp
785                 790                 795                 800

CCA ATG AGG ATG GAT AGG TAT GGT ACC TAT TAT CTT TTA AAA CCG AAA        2448
Pro Met Arg Met Asp Arg Tyr Gly Thr Tyr Tyr Leu Leu Lys Pro Lys
                805                 810                 815

CAA GAG CTT ACA GTC CAA CTG TTC AAG CCT GGC TTA AAC GCA AGA AAC        2496
Gln Glu Leu Thr Val Gln Leu Phe Lys Pro Gly Leu Asn Ala Arg Asn
            820                 825                 830

AAC ATA GCG GAA AAT AAG CAA ATA ACA AAC TTG ACG GCC GGC GTT CCT        2544
Asn Ile Ala Glu Asn Lys Gln Ile Thr Asn Leu Thr Ala Gly Val Pro
        835                 840                 845

GGT GAC GTT GCA TTC TCT GCT CTA GAT GGG AAT AAT TAC ACG CAT TGG        2592
Gly Asp Val Ala Phe Ser Ala Leu Asp Gly Asn Asn Tyr Thr His Trp

850                 855                 860

CAA CCC TTA GAC AAA ATT CAC CGT GCG AAG CTA TTG ATT GAT TTA GGT          2640
Gln Pro Leu Asp Lys Ile His Arg Ala Lys Leu Leu Ile Asp Leu Gly
865                 870                 875                 880

GAA TAC AAC GAG AAA GAG ATT ACC AAG GGA ATG ATT CTT TGG GGG CAG          2688
Glu Tyr Asn Glu Lys Glu Ile Thr Lys Gly Met Ile Leu Trp Gly Gln
                885                 890                 895

AGG CCC GCA AAA AAC ATT TCC ATA TCT ATT TTG CCT CAT TCT GAA AAA          2736
Arg Pro Ala Lys Asn Ile Ser Ile Ser Ile Leu Pro His Ser Glu Lys
            900                 905                 910

GTC GAA AAT TTA TTT GCG AAC GTG ACA GAA ATT ATG CAA AAT TCG GGA          2784
Val Glu Asn Leu Phe Ala Asn Val Thr Glu Ile Met Gln Asn Ser Gly
        915                 920                 925

AAT GAT CAA CTT CTT AAT GAA ACC ATT GGT CAG CTT TTA GAT AAT GCC          2832
Asn Asp Gln Leu Leu Asn Glu Thr Ile Gly Gln Leu Leu Asp Asn Ala
    930                 935                 940

GGA ATT CTT GTC GAG AAC GTT ATT GAT TTT GAT GGC ATA GAA CAA GAG          2880
Gly Ile Leu Val Glu Asn Val Ile Asp Phe Asp Gly Ile Glu Gln Glu
945                 950                 955                 960

GAT GAT GAA TCT TTG GAT GAT GTG CAA GCC TTA TTG CAC TGG AAG AAG          2928
Asp Asp Glu Ser Leu Asp Asp Val Gln Ala Leu Leu His Trp Lys Lys
                965                 970                 975

GAA GAC TTA GCC AAG CTA ATT GAA CAA ATA CCC AGA CTT AAC TTT CTA          2976
Glu Asp Leu Ala Lys Leu Ile Glu Gln Ile Pro Arg Leu Asn Phe Leu
            980                 985                 990

AAA AGA AAA TTT GTG AAA ATT CTG GAT AAC GTG CCA GTG AGC CCA AGT          3024
Lys Arg Lys Phe Val Lys Ile Leu Asp Asn Val Pro Val Ser Pro Ser
        995                 1000                1005

GAG CCA TAC TAC GAA GCA AGT CGC AAC CAG TCG TTA ATC GAG ATA TTA          3072
Glu Pro Tyr Tyr Glu Ala Ser Arg Asn Gln Ser Leu Ile Glu Ile Leu
    1010                1015                1020

CCC AGT AAT AGA ACG ACT TTC ACT ATT GAT TAT GAT AAA TTT GCA GGT          3120
Pro Ser Asn Arg Thr Thr Phe Thr Ile Asp Tyr Asp Lys Phe Ala Gly
1025                1030                1035                1040

GGG TGA                                                                  3126
Gly

(2) INFORMATION FOR SEQ ID NO: 3a:

    (i) SEQUENCE CHARACTERISTICS:
        (A) LENGTH: 1041 amino acids
        (B) TYPE: amino acid
        (D) TOPOLOGY: linear

    (ii) MOLECULE TYPE: protein
    (xi) SEQUENCE DESCRIPTION: SEQ ID NO: 3a:

Met Ile Leu Gly Ser Asn Leu Phe Ser Lys Asn Thr Tyr Ser Arg Gln
  1               5                  10                  15

Pro Tyr Val Ala Asn Gly Tyr Ile Gly Ser Arg Ile Pro Asn Ile Gly
            20                  25                  30

Phe Gly Tyr Ala Leu Asp Thr Leu Asn Phe Tyr Thr Asp Ala Pro Gly
        35                  40                  45

Ala Leu Asn Asn Gly Trp Pro Leu Arg Asn His Arg Phe Ala Gly Ala
    50                  55                  60

Phe Val Ser Asp Phe Tyr Cys Leu Gln Pro Lys Leu Asn Ser Thr Asn
65                  70                  75                  80

Phe Pro Glu Leu Asp Asp Val Gly Tyr Ser Thr Val Ile Ser Ser Ile
                85                  90                  95

Pro Gln Trp Thr Asn Leu Gln Phe Ser Leu Val Asn Asp Ser Lys Trp
            100                 105                 110

Phe Asn Pro Gln Asn Val Thr Leu Asp Asp Val Thr Asn Tyr Ser Gln
        115                 120                 125

Asn Leu Ser Met Lys Asp Gly Ile Val Thr Thr Glu Leu Asp Trp Leu
    130                 135                 140

Asn Ser Gln Ile His Val Lys Ser Glu Ile Trp Ala His Arg His Ile
145                 150                 155                 160

His Pro Leu Gly Val Val Ser Leu Glu Ile Ser Leu Asn Thr Asp His
                165                 170                 175

Leu Pro Ser Asp Phe Asp Ser Leu Asp Val Asn Ile Trp Asp Ile Leu
            180                 185                 190

Asp Phe Asn Thr Ser His Arg Thr Val Leu His Ser Thr Gly Thr Asp
        195                 200                 205

Glu Lys Asn Asn Ala Val Phe Met Ile Val Gln Pro Asp Asn Val Pro
    210                 215                 220

Ser Ser Asn Cys Ala Ile Tyr Ser Thr Cys Thr Val Lys Tyr Glu Asn
225                 230                 235                 240

Ser Thr Asn Pro Ile Asn Ser Ser Glu Ser Phe Glu Glu Lys Asp Val
                245                 250                 255

Ser Ser Asn Ile Tyr Asn Val Ile Leu Thr Glu Asp Gln Pro Lys Ile
            260                 265                 270

Ile Val His Lys Tyr Val Gly Ile Met Ser Thr Glu Phe Asn Lys Asn
        275                 280                 285

Lys Glu Gln Gln Asp Asn Thr Asn Ile Gly Leu Ala Lys Met Ile Ala
    290                 295                 300

Leu Asn Ser Lys Gly Asn Tyr Glu Lys Leu Leu Ser Ser His Lys Arg
305                 310                 315                 320

Ala Trp Tyr Asp Leu Tyr Asn Asp Ala Phe Ile Glu Ile Pro Ser Asp
                325                 330                 335

Ser Leu Leu Glu Met Thr Ala Arg Ser Ser Leu Phe His Leu Leu Ala
            340                 345                 350

Asn Thr Arg Asp Tyr Asn Val Ser Ser Asp Arg Gly Leu Pro Val Gly

        355                 360                 365

Val Ser Gly Leu Ser Ser Asp Ser Tyr Gly Gly Met Val Phe Trp Asp
    370                 375                 380

Ala Asp Ile Trp Met Glu Pro Ala Leu Leu Pro Phe Phe Pro Asn Val
385                 390                 395                 400

Ala Gln Asn Met Asn Asn Tyr Arg Asn Ala Thr His Ser Gln Ala Lys
                405                 410                 415

Leu Asn Ala Glu Lys Tyr Gly Tyr Pro Gly Ala Ile Tyr Pro Trp Thr
            420                 425                 430

Ser Gly Lys Tyr Ala Asn Cys Thr Ser Thr Gly Pro Cys Val Asp Tyr
        435                 440                 445

Glu Tyr His Ile Asn Val Asp Val Ala Met Ala Ser Phe Ser Ile Tyr
    450                 455                 460

Leu Asn Gly His Glu Gly Ile Asp Asp Glu Tyr Leu Arg Tyr Thr Thr
465                 470                 475                 480

Trp Pro Ile Ile Lys Asn Ala Ala Gln Phe Phe Thr Ala Tyr Val Lys
                485                 490                 495

Tyr Asn Ser Ser Leu Gly Leu Tyr Glu Thr Tyr Asn Leu Thr Asp Pro
            500                 505                 510

Asp Glu Phe Ala Asn His Ile Asn Asn Gly Ala Phe Thr Asn Ala Gly
        515                 520                 525

Ile Lys Thr Leu Leu Lys Trp Ala Thr Asp Ile Gly Asn His Leu Gly
    530                 535                 540

Glu Val Val Asp Pro Lys Trp Ser Glu Ile Ser Lys Asp Ile Tyr Ile
545                 550                 555                 560

Pro Arg Ser Ser Ser Asn Ile Thr Leu Glu Tyr Ser Gly Met Asn Ser
                565                 570                 575

Ser Val Glu Ile Lys Gln Ala Asp Val Thr Leu Met Val Tyr Pro Leu
            580                 585                 590

Gly Tyr Ile Asn Asp Glu Ser Ile Leu Asn Asn Ala Ile Lys Asp Leu
        595                 600                 605

Tyr Tyr Tyr Ser Glu Arg Gln Ser Ala Ser Gly Pro Ala Met Thr Tyr
    610                 615                 620

Pro Val Phe Val Ala Ala Ala Ala Gly Leu Leu Asn His Gly Ser Ser
625                 630                 635                 640

Ser Gln Ser Tyr Leu Tyr Lys Ser Val Leu Pro Tyr Leu Arg Ala Pro
                645                 650                 655

Phe Ala Gln Phe Ser Glu Gln Ser Asp Asp Asn Phe Leu Thr Asn Gly
            660                 665                 670

Leu Thr Gln Pro Ala Phe Pro Phe Leu Thr Ala Asn Gly Gly Phe Leu
        675                 680                 685

Gln Ser Asn Leu Phe Gly Leu Thr Gly Ile Arg Tyr Ser Tyr Glu Val
    690                 695                 700

Asp Pro Asp Thr Lys Lys Ile Asn Arg Leu Leu Arg Phe Asn Pro Ile
705                 710                 715                 720

Glu Leu Pro Leu Leu Pro Gly Gly Ile Ala Ile Arg Asn Phe Lys Tyr
                725                 730                 735

Met Asn Gln Val Leu Asp Ile Ile Ile Asp Asp His Asn Gly Thr Ile
            740                 745                 750

Val His Lys Ser Gly Asp Val Pro Ile His Ile Lys Ile Pro Asn Arg
        755                 760                 765

Ser Leu Ile His Asp Gln Asp Ile Asn Phe Tyr Asn Gly Ser Glu Asn
    770                 775                 780

Glu Arg Lys Pro Asn Leu Glu Arg Arg Asp Val Asp Arg Val Gly Asp
785                 790                 795                 800

Pro Met Arg Met Asp Arg Tyr Gly Thr Tyr Tyr Leu Leu Lys Pro Lys
                805                 810                 815

Gln Glu Leu Thr Val Gln Leu Phe Lys Pro Gly Leu Asn Ala Arg Asn
            820                 825                 830

Asn Ile Ala Glu Asn Lys Gln Ile Thr Asn Leu Thr Ala Gly Val Pro
        835                 840                 845

Gly Asp Val Ala Phe Ser Ala Leu Asp Gly Asn Asn Tyr Thr His Trp
    850                 855                 860

Gln Pro Leu Asp Lys Ile His Arg Ala Lys Leu Leu Ile Asp Leu Gly
865                 870                 875                 880

Glu Tyr Asn Glu Lys Glu Ile Thr Lys Gly Met Ile Leu Trp Gly Gln
                885                 890                 895

Arg Pro Ala Lys Asn Ile Ser Ile Ser Ile Leu Pro His Ser Glu Lys
            900                 905                 910

Val Glu Asn Leu Phe Ala Asn Val Thr Glu Ile Met Gln Asn Ser Gly
        915                 920                 925

Asn Asp Gln Leu Leu Asn Glu Thr Ile Gly Gln Leu Leu Asp Asn Ala
    930                 935                 940

Gly Ile Leu Val Glu Asn Val Ile Asp Phe Asp Gly Ile Glu Gln Glu
945                 950                 955                 960

Asp Asp Glu Ser Leu Asp Asp Val Gln Ala Leu Leu His Trp Lys Lys
                965                 970                 975

Glu Asp Leu Ala Lys Leu Ile Glu Gln Ile Pro Arg Leu Asn Phe Leu
            980                 985                 990

Lys Arg Lys Phe Val Lys Ile Leu Asp Asn Val Pro Val Ser Pro Ser
        995                 1000                1005

Glu Pro Tyr Tyr Glu Ala Ser Arg Asn Gln Ser Leu Ile Glu Ile Leu
    1010                1015                1020

Pro Ser Asn Arg Thr Thr Phe Thr Ile Asp Tyr Asp Lys Phe Ala Gly
1025                1030                1035                1040

Gly

(2) INFORMATION FOR SEQ ID NO: 4:

(i) SEQUENCE CHARACTERISTICS:
(A) LENGTH: 804 base pairs
(B) TYPE: nucleic acid
(C) STRANDEDNESS: single
(D) TOPOLOGY: linear

(ii) MOLECULE TYPE: DNA (genomic)

(vi) ORIGINAL SOURCE:
(B) STRAIN: Saccharomyces cerevisiae, wild type

(ix) FEATURE:
(A) NAME/KEY: CDS
(B) LOCATION:1..801

(xi) SEQUENCE DESCRIPTION: SEQ ID NO: 4:

ATG TCG AAA GCT ACA TAT AAG GAA CGT GCT GCT ACT CAT CCT AGT CCT 48
Met Ser Lys Ala Thr Tyr Lys Glu Arg Ala Ala Thr His Pro Ser Pro
1045 1050 1055

GTT GCT GCC AAG CTA TTT AAT ATC ATG CAC GAA AAG CAA ACA AAC TTG 96
Val Ala Ala Lys Leu Phe Asn Ile Met His Glu Lys Gln Thr Asn Leu
1060 1065 1070

TGT GCT TCA TTG GAT GTT CGT ACC ACC AAG GAA TTA CTG GAG TTA GTT 144
Cys Ala Ser Leu Asp Val Arg Thr Thr Lys Glu Leu Leu Glu Leu Val
1075 1080 1085

GAA GCA TTA GGT CCC AAA ATT TGT TTA CTA AAA ACA CAT GTG GAT ATC 192
Glu Ala Leu Gly Pro Lys Ile Cys Leu Leu Lys Thr His Val Asp Ile
1090 1095 1100 1105

TTG ACT GAT TTT TCC ATG GAG GGC ACA GTT AAG CCG CTA AAG GCA TTA 240
Leu Thr Asp Phe Ser Met Glu Gly Thr Val Lys Pro Leu Lys Ala Leu
1110 1115 1120

TCC GCC AAG TAC AAT TTT TTA CTC TTC GAA GAC AGA AAA TTT GCT GAC 288
Ser Ala Lys Tyr Asn Phe Leu Leu Phe Glu Asp Arg Lys Phe Ala Asp
1125 1130 1135

ATT GGT AAT ACA GTC AAA TTG CAG TAC TCT GCG GGT GTA TAC AGA ATA 336
Ile Gly Asn Thr Val Lys Leu Gln Tyr Ser Ala Gly Val Tyr Arg Ile
1140 1145 1150

GCA GAA TGG GCA GAC ATT ACG AAT GCA CAC GGT GTG GTG GGC CCA GGT 384
Ala Glu Trp Ala Asp Ile Thr Asn Ala His Gly Val Val Gly Pro Gly
1155 1160 1165

ATT GTT AGC GGT TTG AAG CAG GCG GCA GAA GAA GTA ACA AAG GAA CCT 432
Ile Val Ser Gly Leu Lys Gln Ala Ala Glu Glu Val Thr Lys Glu Pro
1170 1175 1180 1185

AGA GGC CTT TTG ATG TTA GCA GAA TTG TCA TGC AAG GGC TCC CTA TCT 480
Arg Gly Leu Leu Met Leu Ala Glu Leu Ser Cys Lys Gly Ser Leu Ser
1190 1195 1200

ACT GGA GAA TAT ACT AAG GGT ACT GTT GAC ATT GCG AAG AGC GAC AAA 528

Thr Gly Glu Tyr Thr Lys Gly Thr Val Asp Ile Ala Lys Ser Asp Lys
            1205                1210                1215

GAT TTT GTT ATC GGC TTT ATT GCT CAA AGA GAC ATG GGT GGA AGA GAT     576
Asp Phe Val Ile Gly Phe Ile Ala Gln Arg Asp Met Gly Gly Arg Asp
        1220                1225                1230

GAA GGT TAC GAT TGG TTG ATT ATG ACA CCC GGT GTG GGT TTA GAT GAC     624
Glu Gly Tyr Asp Trp Leu Ile Met Thr Pro Gly Val Gly Leu Asp Asp
    1235                1240                1245

AAG GGA GAC GCA TTG GGT CAA CAG TAT AGA ACC GTG GAT GAT GTG GTC     672
Lys Gly Asp Ala Leu Gly Gln Gln Tyr Arg Thr Val Asp Asp Val Val
1250                1255                1260                1265

TCT ACA GGA TCT GAC ATT ATT ATT GTT GGA AGA GGA CTA TTT GCA AAG     720
Ser Thr Gly Ser Asp Ile Ile Ile Val Gly Arg Gly Leu Phe Ala Lys
                1270                1275                1280

GGA AGG GAT GCT AAG GTA GAG GGT GAA CGT TAC AGA AAA GCA GGC TGG     768
Gly Arg Asp Ala Lys Val Glu Gly Glu Arg Tyr Arg Lys Ala Gly Trp
            1285                1290                1295

GAA GCA TAT TTG AGA AGA TGC GGC CAG CAA AAC TAA                     804
Glu Ala Tyr Leu Arg Arg Cys Gly Gln Gln Asn
        1300                1305

(2) INFORMATION FOR SEQ ID NO: 4a:

        (i) SEQUENCE CHARACTERISTICS:
            (A) LENGTH: 267 amino acids
            (B) TYPE: amino acid
            (D) TOPOLOGY: linear

     (ii) MOLECULE TYPE: protein
     (xi) SEQUENCE DESCRIPTION: SEQ ID NO: 4a:

Met Ser Lys Ala Thr Tyr Lys Glu Arg Ala Ala Thr His Pro Ser Pro
  1               5                  10                  15

Val Ala Ala Lys Leu Phe Asn Ile Met His Glu Lys Gln Thr Asn Leu
             20                  25                  30

Cys Ala Ser Leu Asp Val Arg Thr Thr Lys Glu Leu Leu Glu Leu Val
         35                  40                  45

Glu Ala Leu Gly Pro Lys Ile Cys Leu Leu Lys Thr His Val Asp Ile
     50                  55                  60

Leu Thr Asp Phe Ser Met Glu Gly Thr Val Lys Pro Leu Lys Ala Leu
 65                  70                  75                  80

Ser Ala Lys Tyr Asn Phe Leu Leu Phe Glu Asp Arg Lys Phe Ala Asp
                 85                  90                  95

Ile Gly Asn Thr Val Lys Leu Gln Tyr Ser Ala Gly Val Tyr Arg Ile
            100                 105                 110

Ala Glu Trp Ala Asp Ile Thr Asn Ala His Gly Val Val Gly Pro Gly
        115                 120                 125

Ile Val Ser Gly Leu Lys Gln Ala Ala Glu Glu Val Thr Lys Glu Pro
    130                 135                 140

Arg Gly Leu Leu Met Leu Ala Glu Leu Ser Cys Lys Gly Ser Leu Ser
145                 150                 155                 160

Thr Gly Glu Tyr Thr Lys Gly Thr Val Asp Ile Ala Lys Ser Asp Lys
                165                 170                 175

Asp Phe Val Ile Gly Phe Ile Ala Gln Arg Asp Met Gly Gly Arg Asp
            180                 185                 190

Glu Gly Tyr Asp Trp Leu Ile Met Thr Pro Gly Val Gly Leu Asp Asp
        195                 200                 205

Lys Gly Asp Ala Leu Gly Gln Gln Tyr Arg Thr Val Asp Asp Val Val
    210                 215                 220

Ser Thr Gly Ser Asp Ile Ile Ile Val Gly Arg Gly Leu Phe Ala Lys
225                 230                 235                 240

Gly Arg Asp Ala Lys Val Glu Gly Glu Arg Tyr Arg Lys Ala Gly Trp
                245                 250                 255

Glu Ala Tyr Leu Arg Arg Cys Gly Gln Gln Asn
            260                 265

(2) INFORMATION FOR SEQ ID NO: 5:

(i) SEQUENCE CHARACTERISTICS:
(A) LENGTH: 4292 base pairs
(B) TYPE: nucleic acid
(C) STRANDEDNESS: single
(D) TOPOLOGY: linear

(ii) MOLECULE TYPE: DNA (genomic)

(vi) ORIGINAL SOURCE:
(B) STRAIN: Saccharomyces cerevisiae, wild type

(xi) SEQUENCE DESCRIPTION: SEQ ID NO: 5:

ATGATTTTGG GAAGTAACTT GTTCTCAAAG AATACATACT CGAGACAACC ATATGTTGCT 60

AACGGTTATA TAGGTAGTCG TATTCCCAAT ATTGGGTTCG GCTATGCCTT AGACACCCTG 120

AATTTTTACA CAGACGCACC AGGCGCTTTG AATAACGGTT GGCCCTTAAG AAATCATAGA 180

TTTGCCGGTG CGTTTGTATC GGACTTTTAT TGTCTACAAC CAAAACTAAA TTCAACAAAC 240

TTCCCAGAAT TGGATGATGT AGGATATTCC ACTGTCATTT CATCTATTCC ACAATGGACC 300

AATCTACAGT TCTCATTAGT GAATGATTCT AAGTGGTTCA ATCCACAAAA TGTTACGTTG 360

GATGACGTAA CTAATTATAG CCAAAACTTA TCAATGAAGG ATGGTATCGT AACTACGGAG 420

TTAGATTGGC TAAACAGTCA AATACATGTT AAAAGTGAAA TCTGGGCACA TCGGCACATT 480

CATCCACTGG GAGTGGTTTC TTTGGAAATT TCCCTGAATA CGGACCATTT ACCTTCGGAT 540

TTTGATTCAT TAGATGTTAA TATATGGGAT ATACTTGATT TCAACACATC ACATAGGACT 600

GTTCTACATA GCACGGGAAC AGACGAAAAA AATAATGCGG TTTTCATGAT TGTTCAGCCA 660

GATAACGTTC CATCTTCTAA TTGCGCTATT TACTCAACGT GTACTGTAAA GTATGAAAAT 720
TCCACCAATC CAATAAATTC TAGTGAATCT TTTGAAGAAA AAGATGTTTC TTCTAATATT 780
TATAATGTTA TTTTGACAGA GGACCAACCC AAGATAATCG TTCATAAGTA TGTTGGTATT 840
ATGTCTACTG AGTTCAATAA GAACAAAGAA CAACAAGACA ATACTAATAT TGGTTTGGCC 900
AAAATGATAG CTCTAAACAG TAAAGGCAAT TACGAGAAGC TTTTCAATTC AATTCATCAT 960
TTTTTTTTTA TTCTTTTTTT TGATTTCGGT TTCTTTGAAA TTTTTTTGAT TCGGTAATCT 1020
CCGAACAGAA GGAAGAACGA AGGAAGGAGC ACAGACTTAG ATTGGTATAT ATACGCATAT 1080
GTAGTGTTGA AGAAACATGA AATTGCCCAG TATTCTTAAC CCAACTGCAC AGAACAAAAA 1140
CCTGCAGGAA ACGAAGATAA ATCATGTCGA AAGCTACATA TAAGGAACGT GCTGCTACTC 1200
ATCCTAGTCC TGTTGCTGCC AAGCTATTTA ATATCATGCA CGAAAAGCAA ACAAACTTGT 1260
GTGCTTCATT GGATGTTCGT ACCACCAAGG AATTACTGGA GTTAGTTGAA GCATTAGGTC 1320
CCAAAATTTG TTTACTAAAA ACACATGTGG ATATCTTGAC TGATTTTTCC ATGGAGGGCA 1380
CAGTTAAGCC GCTAAAGGCA TTATCCGCCA AGTACAATTT TTTACTCTTC GAAGACAGAA 1440
AATTTGCTGA CATTGGTAAT ACAGTCAAAT TGCAGTACTC TGCGGGTGTA TACAGAATAG 1500
CAGAATGGGC AGACATTACG AATGCACACG GTGTGGTGGG CCCAGGTATT GTTAGCGGTT 1560
TGAAGCAGGC GGCAGAAGAA GTAACAAAGG AACCTAGAGG CCTTTTGATG TTAGCAGAAT 1620
TGTCATGCAA GGGCTCCCTA TCTACTGGAG AATATACTAA GGGTACTGTT GACATTGCGA 1680
AGAGCGACAA AGATTTTGTT ATCGGCTTTA TTGCTCAAAG AGACATGGGT GGAAGAGATG 1740
AAGGTTACGA TTGGTTGATT ATGACACCCG GTGTGGGTTT AGATGACAAG GGAGACGCAT 1800
TGGGTCAACA GTATAGAACC GTGGATGATG TGGTCTCTAC AGGATCTGAC ATTATTATTG 1860
TTGGAAGAGG ACTATTTGCA AAGGGAAGGG ATGCTAAGGT AGAGGGTGAA CGTTACAGAA 1920
AAGCAGGCTG GGAAGCATAT TTGAGAAGAT GCGGCCAGCA AAACTAAAAA ACTGTATTAT 1980
AAGTAAATGC ATGTATACTA AACTCACAAA TTAGAGCTTC AATTTAATTA TATCAGTTAT 2040
TACCCGGGAA TCTCGGTCGT AATGATTTTT ATAATGACGA AAAAAAAAAA ATTGGAAAGA 2100
AAAAGCTTCT GTCAAGTCAC AAACGTGCGT GGTATGACCT TTACAACGAC GCCTTCATTG 2160
AAATTCCTTC TGACAGTCTT TTAGAAATGA CAGCAAGATC GTCCCTATTC CATTTACTAG 2220
CAAATACAAG AGATTACAAT GTCTCGAGCG ATAGGGGTCT TCCCGTGGGA GTTTCTGGTT 2280
TGTCATCAGA TTCCTATGGT GGTATGGTGT TCTGGGACGC AGATATATGG ATGGAACCTG 2340
CCCTATTGCC TTTCTTCCCA AATGTGGCTC AAAATATGAA TAATTACAGA AATGCTACAC 2400
ATTCGCAGGC AAAGTTAAAT GCAGAGAAAT ATGGATACCC CGGAGCAATA TACCCCTGGA 2460
CATCTGGTAA GTACGCTAAT TGTACTTCTA CGGGACCTTG TGTCGATTAC GAATACCATA 2520
TTAACGTTGA TGTCGCTATG GCCTCCTTTT CCATATACTT GAATGGACAC GAAGGGATTG 2580

ATGACGAGTA TCTGAGATAT ACTACATGGC CAATTATCAA AAACGCAGCC CAATTTTTTA 2640
CTGCTTATGT TAAGTACAAT TCTTCCCTAG GATTGTATGA AACATATAAT TTGACAGATC 2700
CCGACGAGTT TGCTAATCAC ATCAATAACG GGGCTTTCAC GAATGCTGGC ATTAAAACAC 2760
TTCTAAAGTG GGCAACAGAC ATTGGCAATC ATCTCGGCGA GGTCGTTGAC CCCAAATGGA 2820
GTGAAATTTC CAAAGATATT TATATCCCTA GATCCTCATC TAACATCACT TTGGAATATT 2880
CTGGTATGAA TAGCTCAGTG GAGATTAAAC AGGCGGATGT GACTTTAATG GTTTACCCAC 2940
TTGGATATAT CAATGATGAA TCCATATTGA ACAATGCAAT TAAAGATCTT TATTATTATT 3000
CAGAAAGACA GTCTGCGTCT GGGCCTGCAA TGACATATCC GGTTTTTGTG GCCGCAGCTG 3060
CTGGTCTGCT GAATCACGGC TCTTCTTCTC AAAGTTACTT ATATAAATCG GTTCTTCCAT 3120
ACTTACGGGC TCCTTTCGCT CAATTTAGTG AGCAATCAGA CGACAACTTT TTAACAAACG 3180
GATTAACCCA GCCAGCATTC CCCTTTTTAA CAGCTAACGG TGGATTTCTA CAGAGCAATC 3240
TGTTTGGGTT AACAGGAATC CGATATTCTT ATGAGGTTGA TCCAGATACT AAAAAAATTA 3300
ACCGTTTGTT AAGGTTCAAT CCAATAGAAC TACCGTTGCT CCCTGGTGGT ATCGCTATTA 3360
GAAACTTCAA ATATATGAAC CAAGTTTTAG ATATAATAAT TGACGACCAC AATGGTACGA 3420
TTGTTCATAA ATCAGGAGAT GTTCCTATTC ATATAAAGAT ACCAAACAGA TCTCTAATAC 3480
ATGACCAGGA TATCAACTTC TATAATGGTT CCGAAAACGA AAGAAAACCA AATCTAGAGC 3540
GTAGAGACGT CGACCGTGTT GGTGATCCAA TGAGGATGGA TAGGTATGGT ACCTATTATC 3600
TTTTAAAACC GAAACAAGAG CTTACAGTCC AACTGTTCAA GCCTGGCTTA AACGCAAGAA 3660
ACAACATAGC GGAAAATAAG CAAATAACAA ACTTGACGGC CGGCGTTCCT GGTGACGTTG 3720
CATTCTCTGC TCTAGATGGG AATAATTACA CGCATTGGCA ACCCTTAGAC AAAATTCACC 3780
GTGCGAAGCT ATTGATTGAT TTAGGTGAAT ACAACGAGAA AGAGATTACC AAGGGAATGA 3840
TTCTTTGGGG GCAGAGGCCC GCAAAAAACA TTTCCATATC TATTTTGCCT CATTCTGAAA 3900
AAGTCGAAAA TTTATTTGCG AACGTGACAG AAATTATGCA AAATTCGGGA AATGATCAAC 3960
TTCTTAATGA AACCATTGGT CAGCTTTTAG ATAATGCCGG AATTCCTGTC GAGAACGTTA 4020
TTGATTTTGA TGGCATAGAA CAAGAGGATG ATGAATCTTT GGATGATGTG CAAGCCTTAT 4080
TGCACTGGAA GAAGGAAGAC TTAGCCAAGC TAATTGAACA AATACCCAGA CTTAACTTTC 4140
TAAAAAGAAA ATTTGTGAAA ATTCTGGATA ACGTGCCAGT GAGCCCAAGT GAGCCATACT 4200
ACGAAGCAAG TCGCAACCAG TCGTTAATCG AGATATTACC CAGTAATAGA ACGACTTTCA 4260
CTATTGATTA TGATAAATTT GCAGGTGGGT GA 4292